(19) Europäisches Patentamt
European Patent Office
Office européen des brevets

(11) **EP 3 428 646 A1**

(12) **EUROPEAN PATENT APPLICATION**
published in accordance with Art. 153(4) EPC

(43) Date of publication:
**16.01.2019 Bulletin 2019/03**

(51) Int Cl.:
**G01N 33/553** (2006.01)    **G01N 33/543** (2006.01)

(21) Application number: **17763329.4**

(86) International application number:
**PCT/JP2017/009290**

(22) Date of filing: **08.03.2017**

(87) International publication number:
**WO 2017/154989 (14.09.2017 Gazette 2017/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.03.2016 JP 2016045585**

(71) Applicant: **Dai Nippon Toryo Co., Ltd.**
**Konohana-ku**
**Osaka-shi**
**Osaka 554-0012 (JP)**

(72) Inventors:
• **MIYAZAWA, Yuta**
  **Otawara-shi**
  **Tochigi 324-8516 (JP)**
• **MIZOGUCHI, Daigou**
  **Otawara-shi**
  **Tochigi 324-8516 (JP)**

(74) Representative: **Gulde & Partner**
**Patent- und Rechtsanwaltskanzlei mbB**
**Wallstraße 58/59**
**10179 Berlin (DE)**

(54) **COMPOSITION FOR DETECTING SUBSTANCE TO BE TESTED, WHICH CONTAINS GOLD NANOPLATES, AND USE OF SAME**

(57)    The present invention aims to provide a composition which contains gold nanoplates, in particular, suitable for detecting a test substance. A composition of the present invention for detecting a test substance, comprising gold nanoplates, is characterized in that an average aspect ratio of the gold nanoplates (a value obtained by dividing a maximum length of the gold nanoplates by a thickness) is greater than 1 and 10 or less, a concentration of quaternary ammonium cations in the composition is 1 mM or less, and the quaternary ammonium cations are represented by $N^+(R)_4$ (each R is independently selected from linear or branched alkyl groups having 1 to 20 carbon atoms) .

FIG.16

**Description**

Technical Field

The present invention relates to a composition or a freeze-dried product for detecting a test substance, which contains gold nanoplates or gold nanoplates supporting a specific binding substance for the test substance, and a method for detecting a test substance using the same.

Background Art

Light absorption originating from noble metal nanoparticle-based localized surface plasmon resonance (LSPR) is applied to advanced biosensing techniques, and its absorption wavelength depends on the shapes of the nanoparticles, the properties of the metal, and the like. Various metal nanoparticles are studied in this technical field, and gold nanoparticles or gold nanoplates are also drawing attention in the development of highly sensitive biosensors (Patent Literatures 1 to 4 and Non Patent Literatures 1 and 2) . The gold nanoplates have a maximum absorption wavelength in a region from visible light to near-infrared light. Shape control makes it possible to manipulate the position of the maximum absorption wavelength.

Citation List

Patent Literatures

Patent Literature 1: Published Japanese Translation of PCT International Application No. 2015-522828
Patent Literature 2: Japanese Patent No. 4428568
Patent Literature 3: Published Japanese Translation of PCT International Application No. 2008-545884
Patent Literature 4: International Publication No. WO 2006/099312

Non Patent Literatures

Non Patent Literature 1: Chen L., et al., Nano Letters (2014), Vol. 14, No. 12, pp. 7201-7206
Non Patent Literature 2: Scarabelli L, et al., ACS Nano (2014), Vol. 8, No. 6, pp. 5833-5842

Summary of Invention

Problems to be solved by the invention

However, a gold nanoplate which has, for example, both easiness of visual detection and easiness to support a specific binding substance for a test substance, such as an antibody, has not been developed, and the application range of gold nanoplates is not sufficiently wide. The present invention aims to provide a composition which contains gold nanoplates suitable for detecting a test substance in particular.

Means for solving the problems

The present inventors have made earnest studies to solve the above problem and found as a result that, if the average aspect ratio of the gold nanoplates is set within a particular range and the concentration of particular quaternary ammonium cations in the composition is set low, the composition containing the gold nanoplates becomes suitable for detecting a test substance. These findings have led to the completion of the present invention.
Specifically, the present invention provides a composition for detecting a test substance, a freeze-dried product for detecting a test substance, a method for detecting a test substance, and a kit for detecting a test substance, which are described below.

[1] A composition comprising gold nanoplates, for detecting a test substance, wherein
an average aspect ratio of the gold nanoplates (a value obtained by dividing a maximum length of the gold nanoplates by a thickness) is greater than 1 and 10 or less,

a concentration of quaternary ammonium cations in the composition is 0 to 1 mM, and
the quaternary ammonium cations are represented by $N^+(R)_4$ (each R is independently selected from linear or branched alkyl groups having 1 to 20 carbon atoms).

[2] The composition according to [1] described above, wherein
the quaternary ammonium cations are selected from the group consisting of decyltrimethylammonium cations, hexadecyltrimethylammonium cations, and heptadecyltrimethylammonium cations.

[3] The composition according to [1] or [2] described above, not containing a water-soluble polymer having a molecular weight of 500 or more.

[4] The composition according to [1] or [2] described above, further comprising a water-soluble polymer having a molecular weight of 500 or more, wherein a concentration of the water-soluble polymer having the molecular weight of 500 or more in the composition is greater than 0 and 1% by mass or less.

[5] The composition according to [1] or [2] described above, further comprising a water-soluble polymer having a molecular weight of 500 or more, wherein a concentration of the water-soluble polymer having the molecular weight of 500 or more in the composition is greater than 0 and 100 $\mu$M or less.

[6] The composition according to [4] or [5] described above, wherein the gold nanoplates and the water-soluble polymer form a complex.

[7] The composition according to any one of [4] to [6] described above, wherein the water-soluble polymer is selected from the group consisting of poly(sodium styrenesulfonate), polyvinylpyrrolidone, and thiol-terminated polyethylene glycol.

[8] The composition according to any one of [1] to [7] described above, not containing sodium citrate.

[9] The composition according to any one of [1] to [7] described above, further comprising sodium citrate, wherein a concentration of the sodium citrate in the composition is greater than 0 and 50 mM or less.

[10] The composition according to any one of [1] to [9] described above, wherein an average maximum length of the gold nanoplates is less than 100 nm.

[11] The composition according to any one of [1] to [10] described above, wherein the gold nanoplates have a light absorption peak attributed to surface plasmon resonance in a wavelength region of 550 to 740 nm.

[12] The composition according to any one of [1] to [11] described above, exhibiting a color tone of red, magenta, purple, deep blue, blue, cyan, or pale blue.

[13] The composition according to any one of [1] to [12] described above, wherein the gold nanoplates support a specific binding substance for the test substance.

[14] The composition according to [13] described above, wherein a combination of the test substance and the specific binding substance, respectively, is selected from the group consisting of an antigen and an antibody capable of binding thereto, an antibody and an antigen capable of binding thereto, a sugar chain or a glycoconjugate and a lectin capable of binding to the sugar chain or the glycoconjugate, a lectin and a sugar chain or a glycoconjugate capable of binding to the lectin, a hormone or a cytokine and a receptor capable of binding to the hormone or the cytokine, a receptor and a hormone or a cytokine capable of binding to the receptor, a protein and a nucleic acid aptamer or a peptide aptamer capable of binding to the protein, an enzyme and a substrate capable of binding thereto, a substrate and an enzyme capable of binding thereto, biotin and avidin or streptavidin, avidin or streptavidin and biotin, IgG and Protein A or Protein G, Protein A or Protein G and IgG, and a first nucleic acid and a second nucleic acid capable of binding thereto.

[15] A freeze-dried product of the composition according to any one of [1] to [14] described above, for detecting a test substance.

[16] A method for detecting the test substance by using the composition according to [13] or [14] described above or the freeze-dried product according to [15] described above, the method comprising the steps of:

mixing the composition or a resuspension of the freeze-dried product with the test substance to form a complex of the test substance with the gold nanoplates supporting the specific binding substance; and
detecting the complex.

[17] The method according to [16] described above, wherein formation of the complex is detected by means selected from the group consisting of extinction measurement, absorbance measurement, turbidity measurement, particle size distribution measurement, particle diameter measurement, Raman scattering measurement, color-tone change observation, aggregate- or precipitate-formation observation, immunochromatography, electrophoresis, and flow cytometry.

[18] The method according to [16] or [17] described above, further comprising the steps of:
preparing the composition according to [13] or [14] described above by allowing the gold nanoplates in the composition according to any one of [1] to [12] described above to support the specific binding substance for the test substance; and/or resuspending the freeze-dried product according to [15] described above.

[19] A kit for use in the method according to any one of [16] to [18] described above, comprising the composition according to any one of [1] to [14] described above or the freeze-dried product according to [15] described above.

[20] A method for producing the composition according to any one of [1] to [14] described above or the freeze-dried product according to [15] described above, comprising the steps of:

(1) preparing a suspension of gold-nanoplate seed particles; and
(2) preparing a suspension of gold nanoplates.

[21] The method according to [20] described above, wherein the step (1) does not include a step of pre-growing gold-nanoplate seed particles.

Advantageous Effects of Invention

In accordance with the present invention, when gold nanoplates are prepared such that the average aspect ratio of the gold nanoplates (the value obtained by dividing the maximum length of the gold nanoplates by the thickness) is in a range greater than 1 and 10 or less and a composition is prepared such that the concentration of quaternary ammonium cations represented by $N^+(R)_4$ (each R is independently selected from linear or branched alkyl groups having 1 to 20 carbon atoms) is 1 mM or less, it is possible to provide a composition which has a maximum absorption wavelength in the visible light region and which contains gold nanoplates capable of easily supporting a specific binding substance for a test substance such as an antibody. Additionally, as described later, the quaternary ammonium cations could lower the binding property between the gold nanoplates and the specific binding substance for the test substance and have a risk of biotoxicity. For these reason, the composition of the present invention with a reduced concentration of the quaternary ammonium cations is favorable for detecting the test substance.

In addition, the gold nanoplates have a plate shape with a thin thickness and thus have a volume and a mass smaller than those of spherical gold nanoparticles having a particle diameter equal to the average maximum length of the gold nanoplates. Because of this, the gold nanoplates are easily dispersed in a dispersion medium and can disperse with an amount of dispersant smaller than the amount necessary to disperse the spherical gold nanoparticles even when a dispersant is necessary. Moreover, although the specific binding substance for a test substance such as an antibody can also function as a dispersant, it is often expensive. Thus, if the amount used is small, it is possible to reduce the manufacturing cost. Since other dispersants could prevent binding between the gold nanoplates and the specific binding substance for a test substance, a small amount of dispersant in the suspension of the gold nanoplates makes it possible to efficiently support the specific binding substance for a test substance on the gold nanoplates, which can also save the amount used of the specific binding substance for a test substance.

Brief Description of Drawings

Fig. 1 illustrates optical properties of a suspension of gold-nanoplate seed particles.
Fig. 2 illustrates optical properties of suspension A.
Fig. 3 illustrates a SEM observation image of gold nanoplates in the suspension A.
Fig. 4 illustrates optical properties of suspension B (A) and comparison of its extinction spectrum with the suspension A (B).
Fig. 5 illustrates a SEM observation image of gold nanoplates in the suspension B.
Fig. 6 illustrates optical properties of suspension E.
Fig. 7 illustrates a SEM observation image of gold nanoplates in the suspension E.
Fig. 8 illustrates optical properties of suspension H.
Fig. 9 illustrates a SEM observation image of gold nanoplates in the suspension H.
Fig. 10 illustrates optical properties of a suspension of silver-nanoplate seed particles.
Fig. 11 illustrates a SEM observation image of silver-nanoplate seed particles.
Fig. 12 illustrates optical properties of a suspension of silver nanoplates.
Fig. 13 illustrates optical properties of suspension I.
Fig. 14 illustrates a SEM observation image of gold-coated silver nanoplates in the suspension I.
Fig. 15 illustrates optical properties of suspension J.
Fig. 16 illustrates a procedure of an immunochromatographic test.
Fig. 17 illustrates optical properties of suspension K.
Fig. 18 illustrates a SEM observation image of gold nanoplates in the suspension K.
Fig. 19 illustrates optical properties of suspension L.

Fig. 20 illustrates a SEM observation image of gold nanoplates in the suspension L.

Fig. 21 illustrates optical properties of suspension M.

Fig. 22 illustrates a SEM observation image of gold nanoplates in the suspension M.

Fig. 23 illustrates optical properties of suspension N.

Fig. 24 illustrates a SEM observation image of gold nanoplates in the suspension N.

Fig. 25 illustrates optical properties of suspension O (A) and comparison of its extinction spectrum with the suspension K (B).

Fig. 26 illustrates a SEM observation image (A) and a STEM observation image (B) of gold nanoplates in the suspension O.

Fig. 27 illustrates optical properties of suspension P (A) and comparison of its extinction spectrum with the suspension L (B).

Fig. 28 illustrates a SEM observation image (A) and a STEM observation image (B) of gold nanoplates in the suspension P.

Fig. 29 illustrates optical properties of suspension Q (A) and comparison of its extinction spectrum with the suspension M (B).

Fig. 30 illustrates a SEM observation image (A) and a STEM observation image (B) of gold nanoplates in the suspension Q.

Fig. 31 illustrates optical properties of suspension R (A) and comparison of its extinction spectrum with the suspension N (B).

Fig. 32 illustrates a SEM observation image (A) and a STEM observation image (B) of gold nanoplates in the suspension R.

Fig. 33 illustrates optical properties of suspensions S to V.

Fig. 34 illustrates the optical properties of the suspensions S to V corrected such that extinction at a maximum absorption wavelength is 1.0.

Description of Embodiments

Hereinafter, the present invention will be described in more details.

A composition of the present invention for detecting a test substance is characterized by containing gold nanoplates having an average aspect ratio of more than 1 and 10 or less, in which the concentration of quaternary ammonium cations in the composition represented by $N^+(R)_4$ (each R is independently selected from linear or branched alkyl groups having 1 to 20 carbon atoms) is 0 to 1 mM.

The "gold nanoplates" described in the present specification refer to nanoplates (plates) made of gold, and the shape of the upper surface and the lower surface is a polygon such as a triangle, a quadrangle, a pentagon, and hexagon (including a shape with curved corners) or a plate-shaped structure such as a circle. The maximum length may differ for the upper surface and the lower surface of each gold nanoplate. In this case, the longer one is the maximum length of the gold nanoplate. In addition, the value obtained by dividing the maximum length of the nanoplate by the thickness refers to an aspect ratio, which can be used as an index for representing a nanoplate shape. When the shape of a gold nanoplate changes, the position of its maximum absorption wavelength also changes . Thus, the aspect of a gold nanoplate can also be referred to as an index of the maximum absorption wavelength. The average value of the aspect ratios of the gold nanoplates of the present invention (average aspect ratio) is greater than 1 and 10 or less. When the aspect ratio is greater than 1, the gold nanoplate can exhibit a color tone which could not be achieved by a spherical gold nanoparticle. Moreover, if the aspect ratio is greater than 1 and 10 or less, the gold nanoplate has a maximum absorption wavelength in the visible light region or around it. Thus, the gold nanoplate can be used advantageously particularly for a detection method which determines visually whether or not there is a test substance. For example, let the thickness of the gold nanoplate be constant at 10 nm. If the average aspect ratio is greater than 1 and 10 or less, the light absorption peak (maximum absorption wavelength) attributed to surface plasmon resonance in an aqueous suspension can be adjusted in a wavelength region of 550 nm to 740 nm, and if the average aspect ratio is 3.5 to 8.6, the maximum absorption wavelength in the aqueous suspension can be adjusted in a range of 600 nm to 700 nm. The aqueous suspension of gold nanoplates having such a maximum absorption wavelength exhibits a color tone of red, magenta, purple, deep blue, blue, cyan, or pale blue and can control light and shade of the color depending on the concentration of gold nanoplates in the aqueous suspension. Additionally, since the gold nanoplate has a sharp light absorption waveform near the maximum absorption wavelength compared to gold nanoparticles of a cube shape and a rod shape, its suspension exhibits a vivid color tone. In addition, no particular limitation is imposed on the presence of particles other than the gold nanoplates mixed in the suspension. If non-plate-shaped polygonal or spherical gold nanoparticles (maximum absorption wavelength: 540 nm) are mixed, the vividness of the color tone differs depending on the ratio. In order for the suspension of the gold nanoplates to exhibit a vivid color tone, the value obtained by dividing (1) the extinction of 550 nm to 740 nm being the maximum absorption wavelength of the gold nanoplates by (2) the extinction of 540 nm being the maximum absorption wavelength of non-plate-shaped polygonal or spherical gold nanoparticles may be 1.25 or more, preferably 1.5 or more, and further preferably 1.75 or more. The average value of the maximum lengths of the gold nanoplates (average maximum length) may be, but is not particularly limited to, less than 100 nm, preferably 95 to 10 nm, and further preferably 95 to 15 nm, for example, as long as it can be used for detecting a test substance. The maximum length and the thickness of the gold nanoplate may be measured by carrying out, for example, scanning electron microscope (SEM) observation, scanning transmission electron microscope (STEM) observation, or transmission electron microscope (TEM) observation. The maximum length can also be measured by dynamic light scattering particle size distribution measurement apparatus (DLS). Consider the case where the maximum length of the gold nanoplate is measured from a SEM observation image, a STEM observation image, and a TEM observation image. For 100 datasets of 100 arbitrary gold nanoplates produced by measuring the maximum lengths of the particles, 80 average values excluding upper and lower 10% can be used. In this connection, spherical gold nanoparticles are not included in the definition of the gold nanoplate of the present invention since they do not have a plate-shaped structure and the average aspect ratio thereof is 1.

In addition, if the surface charge of the specific binding substance for a test substance such as an antibody, which is to be supported on the surface of the gold nanoplate, is a positive charge, the zeta potential of the gold nanoplates is preferably minus.

The gold nanoplate can control the absorption wavelength and is expected to be applied to treatment, diagnosis, and the like by emitting an electromagnetic wave (light) usable *in vivo*. For example, it is expected to be applied to cancer hyperthermia which uses a photothermal conversion effect for converting absorbed light energy to heat and to photoacoustic imaging which detects an acoustic wave produced when the ambient medium heated by the produced heat expands in volume, i.e. a photoacoustic effect.

The composition of the present invention for detecting a test substance contains the gold nanoplates . The gold content in the composition may be an amount necessary to detect a test substance and may be, for example, 0.000001 to 50% by mass, preferably 0.00001 to 10% by mass, further preferably 0.00005 to 1% by mass, and particularly preferably 0.0001 to 0.1% by mass relative to the total mass of the composition.

The gold content in the composition containing the gold nanoplates may be measured with an ICP emission spectrometer or a UV-visible spectrometer.

In the case of measuring with an ICP emission spectrometer, to be more specific, as illustrated in the following procedure, after a suspension is centrifuged, the supernatant is removed and the resulting precipitate is suspended again in ultrapure water in the same amount as that of the removed supernatant. Then, aqua regia is added to the suspension, followed by boiling. The resulting solution was analyzed by using an ICP emission spectrometer.

1) A suspension of gold nanoplates is centrifuged ($8, 000 \times g$). Then, the supernatant is removed, and the resulting precipitate is suspended again in ultrapure water in the same amount as that of the removed supernatant.
2) Aqua regia is added to the suspension obtained in step 1 above, followed by boiling for 5 minutes. Thereby, gold and silver are dissolved into aqua regia.
3) The solution obtained in step 2 above is measured using an ICP emission spectrometer. The concentration of gold is calculated from a calibration curve which is created by measuring a standard sample of a certain concentration in the same manner as described above.

In the case of measuring with a UV-visible spectrometer, to be more specific, the gold content is calculated by measuring as illustrated in the following procedure.

1) The gold content is calculated by ICP measurement of a gold nanoplate synthetic solution or the amount of gold added.

The gold content in the gold nanoplate synthetic solution before purification (for example, suspensions A, K, and the like of Examples) is measured with an ICP emission spectrometer. Alternatively, a gold content A is calculated supposing that the synthetic yield is 100% based on the total amount of gold added at the time of synthesis of the gold nanoplates.

2) The optical properties of the gold nanoplate synthetic solution are measured with a UV-visible spectrometer.

The gold nanoplate synthetic solution is 4-fold diluted with ultrapure water for measurement with a UV-visible spectrometer.

3) The calculation of the gold content for an extinction of 1.

Suppose that the total value of an extinction B at 540 nm (extinction of non-plate-shaped polygonal or spherical gold nanoparticles) and an extinction C at a maximum absorption wavelength of 550 to 740 nm in the obtained extinction spectrum is the extinction originating from the total amount of gold in the gold nanoplate synthetic solution. Then, the gold content for an extinction of 1 is obtained by the following formula.

```
Gold content for an extinction of 1 (mass%/extinction)

 = A ÷ (B + C)
```

4) The optical properties of a gold nanoplate purification liquid are measured with a UV-visible spectrometer.

The purification liquid for gold nanoplates (for example, suspensions B and O in Examples) is 4-fold diluted with ultrapure water for measurement with a UV-visible spectrometer.

5) The calculation of the gold content.

The total value of an extinction D at 540 nm (extinction of non-plate-shaped polygonal or spherical gold nanoparticles) and an extinction E at a maximum absorption wavelength of 550 to 740 nm in the obtained extinction spectrum is multiplied by the gold content for an extinction of 1 described above to calculate the gold content.

```
Gold content (mass%) = (D + E) × (A ÷ (B + C))
```

In the present specification, a "purity index" of gold nanoplates may be used as an index representing the purity of the gold nanoplates in the composition. The "purity index" described in the present specification refers to a ratio of the non-plate-shaped polygonal or spherical gold nanoparticles to the gold nanoplates in the composition containing the gold nanoplates. To be more specific, the purity index of gold nanoplates is calculated by measuring the composition containing gold nanoplates with a UV-visible spectrometer and dividing the extinction B at 540 nm in the obtained extinction spectrum (originating from the non-plate-shaped polygonal or spherical gold nanoparticles) by the extinction C at the maximum absorption wavelength of 550 to 740 nm (originating from the gold nanoplates).

```
Purity index = B ÷ C
```

It can be said that the purity of gold nanoplates is higher as the value of the purity index is lower. In the composition of the present invention for detecting a test substance, the purity index of gold nanoplates is not limited as long as the detection of a test substance is possible. The purity index may be, for example, 0.05 to 2.00 and preferably 0.10 to 1.0. The purity index of gold nanoplates can be useful in the case of comparing compositions with equal gold contents, average maximum lengths of gold nanoplates, and maximum absorption wavelengths of gold nanoplates.

The composition of the present invention for detecting a test substance can be in the form of a suspension in which the gold nanoplates are suspended in a dispersion medium. Any dispersion medium can be used without particular limitations, as long as it is capable of dispersing the gold nanoplates. For example, the dispersion medium may contain one or more types from water, aqueous buffer solutions (such as a phosphate buffer saline, a Tris-HCl buffer solution, a HEPES buffer solution), alcohols (such as ethanol and methanol), ketones (such as acetone and methyl ethyl ketone), tetrahydrofuran, and the like, and preferably contains water or aqueous buffer solutions favorable in biochemical experiments. In addition, the dispersion medium may contain optional components to be described later which can be contained in the composition of the present invention. The suspension of gold nanoplates may be dispersed in the dispersion medium while left standing, or may be precipitated when left standing but dispersed in the dispersion medium by shaking or ultrasonic dispersion treatment.

In the composition of the present invention for detecting a test substance, the concentration of quaternary ammonium cations represented by $N^+(R)_4$ (each R is independently selected from linear or branched alkyl groups having 1 to 20 carbon atoms) in the dispersion medium is 0 to 1 mM and preferably 0 to 0.5 mM. Further preferably, the quaternary ammonium cations are not contained in the dispersion medium (i.e., the concentration is 0 mM). The quaternary ammonium cations are a compound which is necessary to prepare gold nanoplates having a maximum absorption wavelength in the visible light region but can reduce the binding property between the gold nanoplates described above and a specific binding substance for a test substance to be described later. For this reason, the concentration of the quaternary ammonium cations in the dispersion medium, in particular the concentration of the quaternary ammonium cations when binding the gold nanoplates and the specific binding substance for a test substance is preferably low. In addition, the concentration of the quaternary ammonium cations is preferably low also from the viewpoint of the risk of biotoxicity of the quaternary ammonium cations in the case of using the gold nanoplates *in vivo* such as hyperthermia and photoacoustic imaging described above. The combination of four types of R in the quaternary ammonium cations may be, for example, the combination of linear or branched alkyl groups ($R^a$) having 1 to 9 (preferably 1 to 6 and further preferably 1 to 3) carbon atoms and linear or branched alkyl groups ($R^b$) having 10 to 20 (preferably 12 to 18 and further preferably 14 to 17) carbon atoms, preferably the combination of two types of $R^a$ and two types of $R^b$, and further preferably the combination of three types of $R^a$ and one type of $R^b$. Such a quaternary ammonium cation may be, for example, a decyltrimethylammonium cation, a hexadecyltrimethylammonium cation, or a heptadecyltrimethylammonium cation. As a counter ion of the quaternary ammonium cation, ordinarily employed one as a counter ion of this cation can be used without particular limitation. For example, the chloride ion, the bromide ion, or the iodide ion may be employed.

The concentration of the quaternary ammonium cations in the solvent can be measured and/or calculated without particular limitation by a method ordinarily used in the technical field. For example, the concentration of the quaternary ammonium cations is calculated from the amount of the quaternary ammonium cations used when preparing the gold nanoplate, which is then further multiplied by the dilution factor for the dispersion medium substitution step by e.g. centrifugation. In this way, the concentration of the quaternary ammonium cations in the dispersion medium of the suspension obtained as a result may be calculated. In addition, the quaternary ammonium cations in the dispersion medium may be directly measured by e.g. nuclear magnetic resonance spectroscopy (NMR), mass spectroscopy (MS), or high-performance liquid chromatography (HPLC) to calculate the concentration.

In NMR, the composition of the present invention is dried, and the resulting solid content is dissolved (dispersed) in a deuterated solvent for the measurement. In the deuterated solvent, an internal standard substance (for example, maleic acid) of a known concentration has been added in advance, and the quaternary ammonium cation concentration can be calculated by comparing integrated values of signals derived from the quaternary ammonium cations with integrated values of signals derived from the internal standard substance in the resulting spectrum.

Moreover, in MS, a solution containing quaternary ammonium cations of a known concentration is measured, and a calibration curve is created from a signal intensity derived from the quaternary ammonium cations. Next, when the composition of the present invention is measured, it is possible to calculate the concentration of the quaternary ammonium cations from the obtained detection intensity. For example, in the case of using a time-of-flight mass spectrometer, the concentration of the quaternary ammonium cations can be calculated as follows. A sample containing quaternary ammonium cations of a known concentration is measured with an ionization laser having a certain intensity. After that, the composition of the present invention is measured for comparison of signal intensities. Alternatively, the concentration of the quaternary ammonium cations can be calculated as follows. The composition of the present invention is added with an internal standard substance of a known concentration for comparison between the signal intensity derived from the quaternary ammonium cations and the signal intensity derived from the internal standard substance in the obtained spectrum.

Furthermore, in HPLC, first, multiple solutions of the quaternary ammonium cations of known concentrations are measured, and a calibration curve is created from peak areas derived from the quaternary ammonium cations in the obtained chart. Next, the composition of the present invention is measured. The quaternary ammonium cation concentration can be calculated from the peak areas derived from the quaternary ammonium cations in the obtained chart. For example, it is possible to use Shodex Asahipak GF-310 HQ (manufactured by SHOWA DENKO K.K.) as the separation column for the quaternary ammonium cations and to use a conductivity detector or a differential refractometer for the detection of quaternary ammonium cations.

The composition of the present invention for detecting a test substance may optionally contain or does not have to contain a water-soluble polymer. The "water-soluble polymer" described in the present specification refers to a water-soluble substance having a molecular weight of 500 or more, preferably 500 to 1,000,000, and further preferably 500 to 100,000. The "water-soluble" described in the present specification means that 0.001% by mass or more of the polymer is dissolved in water at normal temperature and normal pressure. The water-soluble polymer used may be polystyrene sulfonic acid or sodium salts thereof, polyvinylpyrrolidone (PVP), polyethylene glycols, polyacrylamides, polyvinyl alcohols, polyacrylic acids, polymethacrylic acids, polyallylamines, dextrans, polymethacrylamides, polyvinylphenols, polyvinylbenzoates, bovine serum albumins (BSA), caseins, or bis(p-sulfonatophenyl)phenylphosphine, and these may be modified with a functional group such as a hydroxy group, a mercapto group, a disulfide group, an amino group, or a carboxyl group, which chemically bond to the gold nanoplates of the present invention. The water-soluble polymer is preferably polystyrene sulfonic acid or sodium salts thereof, PVP, or thiol-terminated polyethylene glycol. Commercially available dispersants which are used in paints, inks, and so forth and contain these water-soluble polymers may be used as the water-soluble polymer to be blended in the composition of the present invention.

The type of the water-soluble polymer contained in the composition of the present invention can be selected depending on the usage of the composition. For example, when the water-soluble polymer is used in an *in vivo* experiment or a cell experiment, polyvinylpyrrolidone, polyethylene glycols, polyvinyl alcohols, and the like, which have favorable biocompatibilities, may be used.

In the case where the composition of the present invention contains the water-soluble polymer, the concentration of the water-soluble polymer dissolved or dispersed in the composition may be, for example, 100 $\mu$M or less, preferably 50 $\mu$M or less, and further preferably 10 $\mu$M or less. Alternatively, the concentration of the water-soluble polymer may be, for example, 1% by mass or less, preferably 0.5% by mass or less, and further preferably 0.1% by mass or less.

Although the present invention is not bound by any particular theory, the dispersion stability of the gold nanoplates improves in the composition when the composition of the present invention is blended with the water-soluble polymer. In addition, when the concentration of the water-soluble polymer is 100 $\mu$M or less or 1% by mass or less, for example, the specific binding substance for a test substance described above is favorably supported by the gold nanoplates, or the gold nanoplates supporting the specific binding substance for a test substance form a stable complex with the test substance compared to the case where the concentration exceeds 100 $\mu$M or 1% by mass; as a result, the sensitivity of detecting the test substance can be enhanced.

The method for measuring the water-soluble polymer concentration includes nuclear magnetic resonance spectroscopy (NMR), size-exclusion chromatography (SEC) (for example, gel-filtration chromatography (GPC)), thermogravimetric-differential thermal analysis (TG-DTA), and the like.

In NMR, the composition of the present invention is dried, and the resulting solid content is dissolved (dispersed) in a deuterated solvent for the measurement. In the deuterated solvent, an internal standard substance (for example, maleic acid) of a known concentration has been added in advance, and the water-soluble polymer concentration can be calculated by comparing integrated values of signals derived from the water-soluble polymer with integrated values of signals derived from the internal standard substance in the resulting spectrum.

Moreover, in GPC, first, multiple aqueous solutions of the water-soluble polymer of known concentrations are analyzed, and a calibration curve is created from peak areas derived from the water-soluble polymer in the obtained chart. Next, the composition of the present invention is measured. The water-soluble polymer concentration can be calculated from the peak areas derived from the water-soluble polymer in the obtained chart.

Further, in TG-DTA, the amount of the water-soluble polymer can be calculated from a change in weight when the composition of the present invention is measured for the dried solid content.

The composition of the present invention for detecting a test substance may optionally contain or does not have to contain sodium citrate. If sodium citrate is contained, the dispersibility of the gold nanoplates in the composition improves. If the composition contains sodium citrate, the concentration of sodium citrate in the composition may be, for example, 50 mM or less, preferably 25 mM or less, and further preferably 13 mM or less. If the concentration of sodium citrate is 50 mM or less, the gold nanoplates in the composition are unlikely to aggregate.

The composition of the present invention may contain an optional component, as long as the gold nanoplates are not adversely influenced. Such an optional component includes, for example, reagents (for example, sodium borohydride and ascorbic acid) used when producing the gold nanoplates, dispersants (for example, polyethylene glycols having a molecular weight of less than 500), and the like.

The composition of the present invention for detecting a test substance can be used to label a specific binding substance for a test substance, that is, a detection reagent. In other words, the gold nanoplates of the present invention can support a specific binding substance for a test substance. The "support" described in the present specification means that the gold nanoplates are linked to a specific binding substance for a test substance to form a complex, regardless of the linking mode such as a covalent bond, a non-covalent bond, or direct or indirect linking. As the supporting method, normal supporting methods can be employed without particular limitations. It is possible to employ: a method in which the gold nanoplates are directly linked to a specific binding substance for a test substance by utilizing physical adsorption, chemical adsorption (covalent bond to the surface), chemical bond (covalent bond, coordinate bond, ionic bond, or metallic bond), or the like; and a method in which a portion of the above-described water-soluble polymer is linked to the surfaces of the gold nanoplates, and then a specific binding substance for a test substance is directly or indirectly linked to an end, or a main chain or a side chain, of the water-soluble polymer. For example, in a case where the specific binding substance for the test substance is an antibody, the gold nanoplates of the present invention are mixed with a solution of the antibody, shaken, and centrifuged, so that the gold nanoplates supporting the antibody (labeled detection reagent) can be obtained as a precipitate. In addition, in the case of the gold nanoplates of the present invention support an antibody by electrostatic adsorption, the support efficiency of the antibody can be improved if the surfaces of the gold nanoplates are coated with polystyrene sulfonic acid having a minus charge. In this connection, the specific binding substance for a test substance is also favorable particularly as a dispersant for the gold nanoplates supporting the specific binding substance for a test substance because it can improve the dispersion stability of the gold nanoplates in the composition of the present invention being a suspension in the same manner as the water-soluble polymer described above.

As the specific binding substance for the test substance of the present invention, any specific binding substance can be used without particular limitations, as long as it is capable of detecting a detection target of a test substance through complex formation with the test substance and capable of utilizing the gold nanoplates as a label. Specific examples of a combination of the test substance and the specific binding substance for test substance include an antigen and an antibody capable of binding thereto, a sugar chain or a glycoconjugate and a lectin capable of binding to the sugar chain or the glycoconjugate, a lectin and a sugar chain or a glycoconjugate capable of binding to the lectin, a hormone or a cytokine and a receptor capable of binding to the hormone or the cytokine, a receptor and a hormone or a cytokine capable of binding to the receptor, a protein and a nucleic acid aptamer or a peptide aptamer capable of binding to the protein, an enzyme and a substrate capable of binding thereto, a substrate and an enzyme capable of binding thereto, biotin and avidin or streptavidin, avidin or streptavidin and biotin, IgG and Protein A or Protein G, Protein A or Protein G and IgG, a first nucleic acid and a second nucleic acid capable of binding (hybridizing) thereto, and the like. The second nucleic acid may be a nucleic acid containing a sequence complementary to that of the first nucleic acid.

In the case where the test substance is an antigen, the specific binding substance for the antigen may be an antibody. The antibody may be a polyclonal antibody, a monoclonal antibody, a single chain antibody, or fragments thereof, all of which are capable of specifically binding to the antigen. The fragments may be a F(ab) fragment, a F(ab') fragment, a F(ab')$_2$ fragment, or a F(v) fragment. The antigen serving as a test substance may be a lectin such as concanavalin A (ConA), wheat germ agglutinin, and ricin; may be a virus such as *influenza viruses,* adenoviruses, RS virus, *rotaviruses, human papillomaviruses, human immunodeficiency viruses, hepatitis B virus,* Zika virus, and dengue virus or substances thereof (for example, *hepatitis B virus* antigens (HBs antigens) or influenza virus hemagglutinins); may be a pathogenic microorganism such as *Aspergillus flavus, Chlamydia, Treponema pallidum, Streptococcus hemolyticus, Bacillus anthracis, Staphylococcus aureus, Shigella, Escherichia coli, Salmonella, Salmonella typhimurium, Salmonella* paratyphi A, *Pseudomonas aeruginosa,* and *Vibrio parahaemolyticus,* or substances thereof (for example, *Aspergillus flavus* aflatoxin (B1, B2, G1, G2, M1, or the like), enterohemorrhagic *Escherichia coli* verotoxin, or *Streptococcus hemolyticus* streptolysin O) ; may be a blood protein such as immunoglobulin G (IgG), rheumatoid factor, and C-reactive protein (CRP) ; may be a glycoprotein such as mucins; may be a hormone such as insulin or a pituitary hormone (for example, growth hormone (GH), adrenocorticotropic hormone (ACTH), thyroid-stimulating hormone (TSH), follicle-stimulating hormone (FSH), luteinizing hormone (LH), prolactin, or melanocyte-stimulating hormone (MSH)), a thyrotropin-releasing hormone (TRH), a thyroid hormone (for example, diiodothyronine or triiodothyronine), a chorionic gonadotropin, a calcium metabolism regulating hormone (for example, calcitonin or parathormone), a pancreatic hormone, a gastrointestinal hormone, a vasoactive intestinal peptide, a follicle hormone (for example, estrone), a natural or synthetic corpus luteum hormone (for example, progesterone), a male sex hormone (for example, testosterone), and an adrenal cortical hormone (for example, cortisol) ; may be other *in vivo* substances such as serotonin, urokinase, ferritin, substance P, prostaglandins, and cholesterols; may be a tumor marker such as prostatic acid phosphatase (PAP), prostate-specific antigen (PSA), alkaline phosphatase, transaminase, trypsin, pepsinogen, $\alpha$-fetoprotein (AFP), and carcinoembryonic antigen (CEA) ; may be a sugar chain antigen such as a blood group antigen; may be a marker, such as hemoglobin and transferrin, used in the detection of fecal occult blood; or may be a D-dimer which is a type of a fibrin degradation product produced when, in a blood coagulation and fibrinolysis system, an action of an active type factor XIII causes cross-linking of a stabilized fibrin and the stabilized fibrin is then degraded by plasmin. Moreover, in the case where the antigen serving as a test substance is an *in vivo* substance such as a hormone or a cytokine, the specific binding substance for the *in vivo* substance may be not only an antibody but also a receptor. In the case where the antigen serving as a test substance is a sugar chain or a glycoconjugate having a sugar chain, the specific binding substance for the sugar chain or the glycoconjugate having a sugar chain may be not only an antibody but also a lectin. Additionally, the antigen serving as a test substance may be a hapten such as penicillin and cadmium.

In the case where the test substance is an antibody, the specific binding substance for the antibody may be an antigen. The antigen may be a complete antigen or a fragment thereof which are capable of specifically binding to the antibody, or may be a fusion substance in which a complete antigen and a fragment thereof binds to another carrier. The antibody serving as a test substance may be an autoantibody such as an anti-cyclic citrullinated peptide (CCP) antibody or an anti-phospholipid antibody, or may be an antibody against an exogenous antigen, such as an anti-Chlamydia antibody, an anti-HIV antibody, or an anti-HCV antibody.

In the case where the test substance is a sugar chain, the specific binding substance for the sugar chain may be a lectin. The lectin may be a galectin, a C-type lectin, a legume lectin, or fragments thereof, all of which are capable of specifically binding to the sugar chain. The sugar chain serving as a test substance may be a monosaccharide or a polysaccharide, or may be a glycoconjugate in which a monosaccharide or a polysaccharide is linked to a protein or a lipid. For example, in a case where the test substance is a sugar chain containing mannose, a legume lectin concanavalin A (ConA) can be used as the specific binding substance for the sugar chain.

In the case where the test substance is a lectin, the specific binding substance for the lectin may be a sugar chain. The sugar chain may be a monosaccharide, a polysaccharide, or a glycoconjugate, all of which are capable of specifically binding to the lectin, or a fusion substance in which a monosaccharide, a polysaccharide, or a glycoconjugate binds to another carrier. The lectin serving as a test substance may be a galectin, a C-type lectin, or a legume lectin. For example, in a case where the test substance is a legume lectin concanavalin A (ConA), a sugar chain containing mannose can be used as the specific binding substance for the lectin.

In the case where the combination of the test substance and the specific binding substance for the test substance is a protein and a nucleic acid aptamer or a peptide aptamer capable of binding to the protein, the nucleic acid aptamer may be, for example, a DNA aptamer capable of binding to: a bacterium such as *Bacillus anthracis, Staphylococcus aureus, Shigella sonnei, Escherichia coli, Salmonella typhimurium, Streptococcus hemolyticus, Salmonella* paratyphi A, Staphylococcal enterotoxin B, *Pseudomonas aeruginosa,* or *Vibrio parahaemolyticus;* a tumor marker expressed on the epithelial cell surface, such as mucin 1; or an enzyme such as β-galactosidase or thrombin; or the like. Alternatively, the nucleic acid aptamer may be an RNA aptamer capable of binding to a Tat protein or a Rev protein of *human immunodeficiency virus.* The peptide aptamer may be, for example, a peptide aptamer capable of binding to an oncoprotein HPV16 E6 of *human papillomavirus* (HPV).

In a case where the test substance is a vitamin, the specific binding substance for the vitamin may be a vitamin binding protein such as transcalciferin capable of binding to vitamin D and a transcobalamin capable of binding to vitamin B12. In a case where the test substance is an antibiotic, the specific binding substance for the antibiotic may be a penicillin-binding protein such as PBP1 and PBP2 capable of binding to penicillin.

The nucleic acid serving as a test substance or a substance capable of binding to the test substance may be, for example, a DNA, an RNA, an oligonucleotide, a polynucleotide, or amplification products thereof.

In an embodiment, the present invention relates to a freeze-dried product for detecting a test substance, and the freeze-dried product is a freeze-dried product of the composition for detecting the test substance. The freeze-dried product of the present invention can be prepared from the composition for detecting the test substance without being particularly limited by the method ordinarily used in the technical field. The freeze-dried product of the present invention is an embodiment favorable for carrying or storing a substance for detecting the test substance. The freeze-dried product of the present invention can be used by being suspended again in a dispersion medium at the time of detecting a test substance. As the dispersion medium for the freeze-dried product of the present invention, it is possible to use without particular limitation the above-described dispersion medium which can be used for the composition for detecting the test substance.

In an embodiment, the present invention also relates to a method for detecting a test substance. The gold nanoplates supporting a specific binding substance for the test substance can bond to the corresponding test substance and detect it. The method for detecting the test substance of the present invention includes: the step of mixing the composition of the present invention or the resuspension of the freeze-dried product of the present invention with the test substance to form a complex of the test substance and the gold nanoplates supporting the specific binding substance for the test substance; and the step of detecting the complex. The gold nanoplates supporting the specific binding substance for the test substance of the present invention may be prepared immediately before carrying out the method for detecting the test substance of the present invention. In other words, the method for detecting the test substance of the present invention may further include the step of allowing the gold nanoplates to support the specific binding substance for the test substance. In addition, the method for detecting the test substance of the present invention may further include the step of resuspending the freeze-dried product of the present invention.

The complex can be detected without particular limitations by utilizing means normally used in the field of detecting test substances or means normally used to detect aggregates or precipitates. For example, formation of the complex may be detected by means selected from the group consisting of extinction measurement, absorbance measurement, turbidity measurement, particle size distribution measurement, particle diameter measurement, Raman scattering measurement, color-tone change observation, aggregate- or precipitate formation observation, immunochromatography, electrophoresis, and flow cytometry.

Absorbance refers to the strength of light absorption of an object when parallel light rays transmit through the object (absorbance in the narrow sense). In an actual measurement, it is necessary to take into account light loss attributed to reflection, scattering, or the like on the surface of the object (absorbance in the broad sense) . The broad sense absorbance, which means the intensity of light loss attributed to various factors such as absorption, reflection, and scattering of light, refers to extinction particularly in the present specification. When a complex of the test substance and the gold nanoplates supporting the specific binding substance for the test substance is formed, the extinction or the absorbance in a wavelength region around the maximum absorption wavelength of the gold nanoplates decreases.

The extinction measurement or the absorbance measurement may be carried out in a wavelength region of a range of 200 to 2500 nm or may be carried out at the maximum absorption wavelength of the gold nanoplates having a range of 430 to 2000 nm. The extinction or the absorbance may be measured with a UV-visible spectrometer (for example, UV-Vis-NIR spectrometer MPC 3100 UV-3100 PC manufactured by Shimadzu Corporation) or the like ordinarily used for these measurements.

When a complex of the test substance and the gold nanoplates supporting the specific binding substance for the test substance is formed, the gold nanoplates are cross-linked to each other via the test substance, resulting in a particle diameter larger than that of a single gold nanoplate. When the particle diameter of the particles dispersed in the composition of the present invention becomes large, the absorption, reflection, or scattering of light incident on the composition increases, and the composition exhibits muddiness. The absorption, reflection, or scattering of light in the composition exhibiting muddiness can be measured as an extinction or an absorbance. The extinction or the absorbance which increases depending on the degree of muddiness is also referred to as turbidity.

In the composition of the present invention, if multiple particles are present adjacent to each other when a complex of the test substance and the gold nanoplates supporting the specific binding substance for the test substance is formed, there exists a wavelength region in which the extinction or the absorbance increases depending on the formation of the complex on the long-wavelength side of the maximum absorption wavelength of the gold nanoplates due to the dipole-dipole interaction (Nano Lett., Vol. 4, No. 9, (2004), p. 1627-1631). The complex may be detected by measuring the extinction or the absorbance in this wavelength region as the turbidity. For example, if the maximum absorption wavelength of the gold nanoplates is 560 nm, turbidity measurement may be carried out in a wavelength region of 660 to 840 nm, and if the maximum absorption wavelength of the gold nanoplates is 620 nm, turbidity measurement may be carried out in a wavelength region of 720 to 900 nm. The turbidity may be measured with a UV-visible spectrometer (for example, UV-Vis-NIR spectrometer MPC 3100 UV-3100 PC manufactured by Shimadzu Corporation), a turbidity meter, or the like ordinarily used for the measurement.

In immunochromatography, gold nanoplates gather at a detection site (in the form of line) . In this event, when the gold nanoplates absorb light in the visible region, the formation of the complex can be recognized as a color. The result of this test can be quantified by visual judgment and luminance difference analysis. In the visual judgment, whether or not the detection line is present after the immunochromatographic test is visually checked, and the lowest concentration of a test substance at which the detection line can be checked may be regarded as the detection sensitivity. In the luminance analysis, when an absolute value of a value obtained by subtracting a luminance difference 2 from a luminance difference 1, which are as described below, is 2 (detection limit luminance difference) or larger, the lowest concentration of a test substance may be regarded as the detection sensitivity.

1. The luminance difference between a detection line and a section other than the detection line (control region) when an immunochromatographic test is conducted using a developing solution containing no test substance.
2. The luminance difference between a detection line and a section other than the detection line (control region) on an immunochromatographic support when an immunochromatographic test is conducted using a developing solution containing a test substance.

Meanwhile, in an immunochromatographic test using gold nanoplates which absorb light in the near-infrared region, the detection portion is irradiated with near-infrared light after the test. The light absorption in this event is measured, so that the detection can be checked.

The gold nanoplates have a plate shape with a thin thickness and thus have a volume and a mass smaller than those of spherical gold nanoparticles having a particle diameter equal to the average maximum length of the gold nanoplates. Because of this, the gold nanoplates in the composition of the present invention (gold nanoplates supporting the specific binding substance for the test substance) exhibit a good mobility (developability) on the immunochromatographic support, making it possible to increase the luminance difference between the detection line and the section other than the detection line (control region) compared to the case of using spherical gold nanoparticles. In addition, since the gold nanoplates in the composition of the present invention (gold nanoplates supporting the specific binding substance for the test substance) develop rapidly on the immunochromatographic support, it is possible to shorten the time required for the determination (detection) of the test substance. Plus, in general, the background color of the immunochromatographic support appears whiter in the case of pale rather than perfect white, which emphasizes the color of the detection line. When gold nanoplates exhibiting blue are used in a white immunochromatographic support, the support turns white with slight blue even when the color of the gold nanoplates remains in the support after development. Thus, it is possible to significantly improve the visibility of the detection line compared to the case of using spherical gold nanoparticles which can be prepared only in red.

The test substance can be detected in a highly sensitive manner by using the composition of the present invention for detecting a test substance. Thus, it is possible to suppress the amount of the specific substance for a test substance used. Since the specific substance for a test substance such as an antibody is expensive, it is possible to reduce the cost for detecting a test substance by reducing the amount of the specific substance for a test substance used in accordance with the present invention.

In an embodiment, the present invention relates to a kit for use in the method for detecting the test substance, including the composition for detecting the test substance or the freeze-dried product for detecting the test substance. The kit of the present invention may further contain a standard product of the test substance, an immunochromatographic test paper, or a manual describing a use method.

In an embodiment, the present invention relates to a method for producing a composition for detecting the test substance or a freeze-dried product for detecting the test substance, including (1) the step of preparing a suspension of gold-nanoplate seed particles and (2) the step of preparing a suspension of gold nanoplates. The step (1) may include a two-stage step of the step of pre-growing gold-nanoplate seed particles and the step of growing the same, or may start the step of first growing the gold-nanoplate seed particles without including the step of pre-growing. In the case of pre-growing and then growing the gold-nanoplate seed particles, seed particles are efficiently reared (pre-grown) under a condition of a low concentration (for example, 15 mM) of quaternary ammonium cations such as hexadecyltrimethylammonium chloride, and then the concentration of the quaternary ammonium cations is set high (for example, 50 mM) to reduce the particle growth rate. In this way, the seed particles are reared (grown) in a uniform manner. In the case of first growing the gold-nanoplate seed particles without pre-growing, it is possible to more easily produce the seed particles because it is unnecessary to change the concentration of the quaternary ammonium cations at a certain midpoint. The shapes and the sizes of the seed particles are likely to be the same with the pre-growing step. However, without the step, it is possible to produce a composition for detecting the test substance, the method for detecting the test substance, and seed particles provided with a sufficient uniformity necessary to produce gold nanoplates to be used for the kit. Purification methods for removing non-plate-shaped polygonal or spherical gold nanoparticles (maximum absorption wavelength: 540 to 550 nm) which can be generated during production of the gold nanoplates include a method for selectively precipitating the gold nanoplates by increasing the concentration of the quaternary ammonium cations in the suspension (depletion flocculation), ultrafiltration of a purification method using the difference in particle sizes (for example, ultrafiltration by tangential flow filtration), size-exclusion chromatography (for example, gel-filtration chromatography), density gradient centrifugation, and the like.

In addition, the concentration of quaternary ammonium cations in the dispersion medium of the suspension of gold nanoplates prepared from the seed particles can be reduced by substituting the dispersion medium with a dispersion medium not containing quaternary ammonium cations by, for example, centrifugation, dialysis, size-exclusion chromatography (for example, gel-filtration chromatography), and/or ultrafiltration (for example, ultrafiltration by tangential flow filtration). For example, consider the case of reducing the concentration of quaternary ammonium cations by centrifugation. Specifically, the suspension of gold nanoplates is centrifuged to remove the supernatant. After the measurement of the mass of the obtained precipitate, the precipitate is suspended again using a dispersion medium (not containing quaternary ammonium cations) in the same amount as that of the removed supernatant. For example, suppose that the mass of the precipitate is 1 g and the mass of the dispersion medium used for the resuspension is 99 g. Then, the concentration of quaternary ammonium cations in the dispersion medium of the suspension containing gold nanoplates is 100-fold diluted.

Hereinafter, the present invention will be described specifically by way of Examples. However, the scope of the present invention is not limited to these Examples.

EXAMPLES

1. Suspension Containing Gold Nanoplates

(1) Preparation of Suspension of Gold-Nanoplate Seed Particles

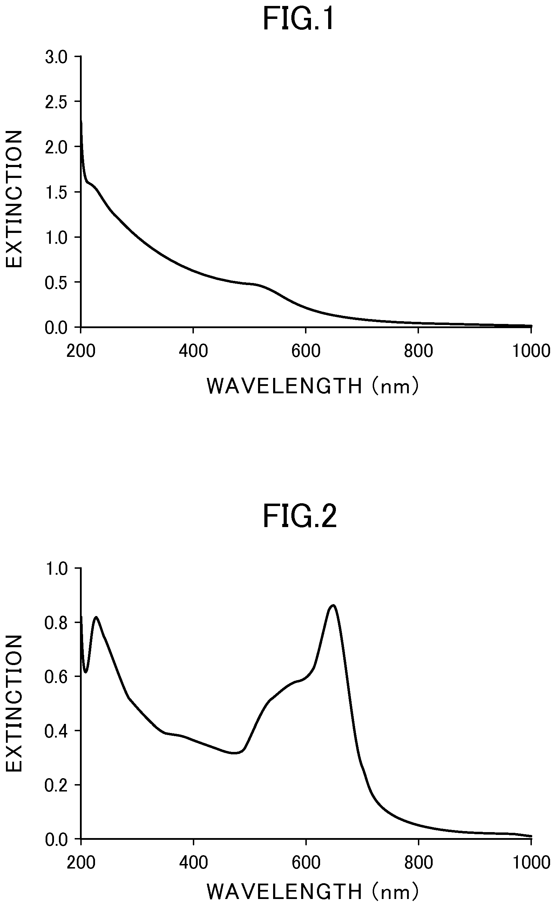

Into 24 g of 100 mM aqueous solution of hexadecyltrimethylammonium chloride (CTAC), 0.13 g, of 50 mM aqueous solution of chloroauric acid and 1.5 g of 10 mM aqueous solution of sodium borohydride were added under stirring. The obtained solution was left standing in an incubator (30°C) for 120 minutes to prepare a gold-nanoplate seed particle suspension. Fig. 1 shows the optical properties of the prepared suspension (raw solution). The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm.

The gold content of the gold-nanoplate seed particle suspension was 0.0049% by mass relative to the total mass of the suspension. Fig. 1 shows the optical properties of this suspension.

(2) Preparation of Suspension of Gold Nanoplates

(2-1) Synthesis not Including Pre-Growing

(2-1-1) Preparation of Suspension of Gold Nanoplates

(Suspension A)

A strong stirrer ($\varphi 8 \times 38$ mM) was placed in 36 g of 50 mM aqueous solution of CTAC and was stirred at 500 rpm, followed by addition of 0.5 g of 50 mM aqueous solution of chloroauric acid, 0.3 g of 10 mM aqueous solution of sodium iodide, and 0.4 g of 100 mM aqueous solution of L-ascorbic acid. After 30-second stirring, 0.3 g of 10-fold diluted solution of gold-nanoplate seed particle suspension was added, followed by stirring for 5 seconds. The obtained suspension was left standing in an incubator (30°C) for 12 hours to prepare a suspension of gold nanoplates (suspension A). When proportional calculation was carried out using the dilution ratio from the aqueous solution of CTAC used in the preparation of this suspension (in this case, the specific gravity of each solution was assumed to be 1, and the amount of CTAC in the 10-fold diluted solution of the gold-nanoplate seed particle suspension was ignored), the CTAC concentration in the dispersion medium of the suspension A was determined to be 48.0 mM (the same calculation method was employed in other Examples unless otherwise noted).

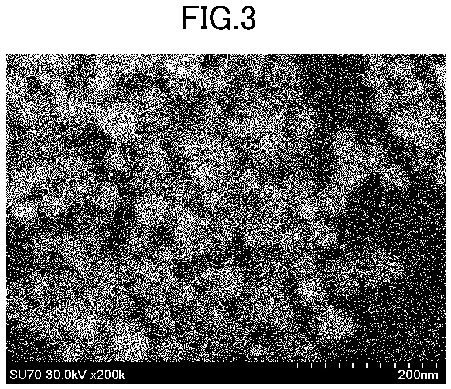

When the suspension A was 4-fold diluted by volume with ultrapure water, the color tone of the suspension was blue. Fig. 2 shows the optical properties of this 4-fold diluted suspension. The wavelength exhibiting the maximum absorbance was 648 nm (extinction: 0.86). Since the extinction at 540 nm being the maximum absorption wavelength of non-plate-shaped polygonal or spherical gold nanoparticles was 0.52, the purity index of gold nanoplates was calculated to be 0.60. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 3 shows the results of scanning electron microscope (SEM) observation of the gold nanoplates in the obtained suspension A with an ultra-high-resolution analytical scanning electron microscope SU-70 (manufactured by Hitachi, Ltd.). SEM observation revealed that the gold nanoplates had a maximum length of 50 nm, a thickness of 24 nm, and an aspect ratio of 2.1.

The pH of the suspension A as a result of measurement at room temperature (20°C) was 2. 4. For the pH measurement, a Twin pH Meter B-212 manufactured by HORIBA, Ltd. was used (glass electrode method).

The gold content of the suspension A was 0.0131% by mass relative to the total mass of the suspension.

(2-2) Synthesis Including Pre-Growing

(2-2-1) Preparation of Suspension of Gold Nanoplates

(Suspension K)

Stirring was carried out on 3.16 g of 15 mM aqueous solution of CTAC, followed by addition of 0.004 g of 20 mM aqueous solution of sodium iodide, 0.013 g of 50 mM aqueous solution of chloroauric acid, and 0.013 g of 100 mM aqueous solution of L-ascorbic acid to prepare a pre-growing solution. Next, stirring was carried out on 40 g of 51 mM aqueous solution of CTAC, followed by addition of 0.15 g of 20 mM aqueous solution of sodium iodide, 0.5 g of 50 mM aqueous solution of chloroauric acid, and 0.4 g of 100 mM aqueous solution of L-ascorbic acid to prepare a growing solution. The pre-growing solution was added with 0.031 g of 10-fold diluted solution of gold-nanoplate seed particle suspension to prepare a pre-growing gold nanoplate suspension. The total amount of pre-growing gold nanoplate suspension was added to the growing solution. The obtained suspension was left standing in an incubator (30°C) for 12 hours to prepare a suspension of gold nanoplates (suspension K) . The CTAC concentration in the dispersion medium of the suspension K was calculated to be 47.2 mM.

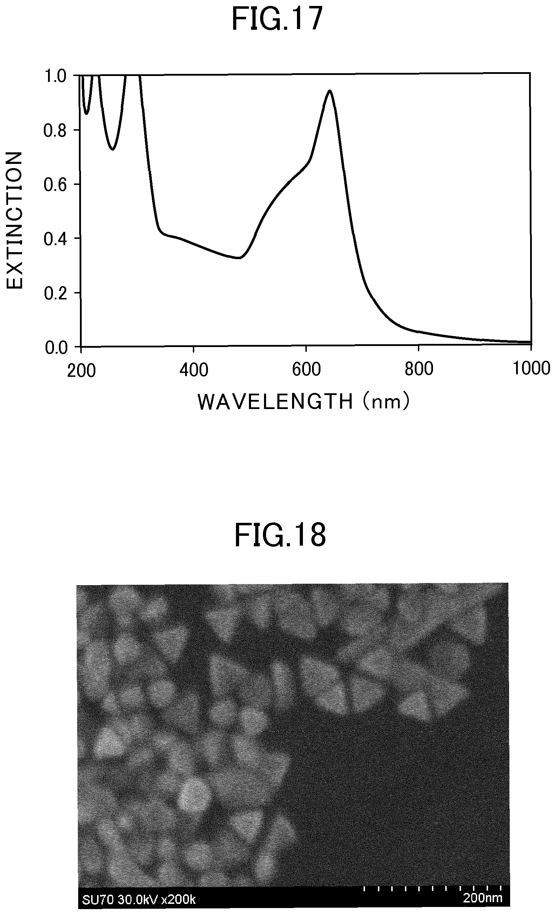

When the suspension K was 4-fold diluted by volume with ultrapure water, the color tone of the suspension was blue. Fig. 17 shows the optical properties of this 4-fold diluted suspension. The wavelength exhibiting the maximum absorbance was 644 nm (extinction: 0.94) . Since the extinction at 540 nm being the maximum absorption wavelength of non-plate-shaped polygonal or spherical gold nanoparticles was 0.53, the purity index of gold nanoplates was calculated to be 0.56. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 18 shows the results of scanning electron microscope (SEM) observation of the gold nanoplates in the obtained suspension K with an ultra-high-resolution analytical scanning electron microscope SU-70 (manufactured by Hitachi, Ltd.). In addition, the pH of the suspension K as a result of measurement at room temperature (20°C) was 2.5. For the pH measurement, a Twin pH Meter B-212 manufactured by HORIBA, Ltd. was used (glass electrode method) .

The gold content of the suspension K was 0.0114% by mass relative to the total mass of the suspension.

(2-2-2) Preparation of Suspension of Gold Nanoplates

(Suspension L)

A suspension of gold nanoplates (suspension L) was prepared in the same manner as in the suspension of gold nanoplates (suspension K) except that the above-described amount of 10-fold diluted solution of gold-nanoplate seed particle suspension added was changed from 0.031 g to 0.016 g. The CTAC concentration in the dispersion medium of the suspension L was calculated to be 47.1 mM.

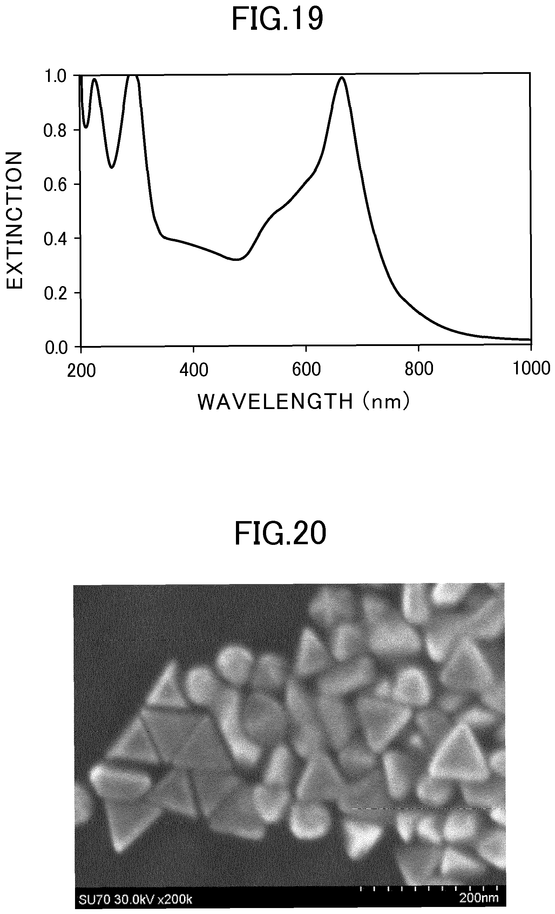

When the suspension L was 4-fold diluted by volume with ultrapure water, the color tone of the suspension was blue. Fig. 19 shows the optical properties of this 4-fold diluted suspension. The wavelength exhibiting the maximum absorbance was 668 nm (extinction: 0.99) . Since the extinction at 540 nm being the maximum absorption wavelength of non-plate-shaped polygonal or spherical gold nanoparticles was 0.48, the purity index of gold nanoplates was calculated to be 0.48. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 20 shows the results of scanning electron microscope (SEM) observation of the gold nanoplates in the obtained suspension L with an ultra-high-resolution analytical scanning electron microscope SU-70 (manufactured by Hitachi, Ltd.). In addition, the pH of the suspension L as a result of measurement at room temperature (20°C) was 2.5. For the pH measurement, a Twin pH Meter B-212 manufactured by HORIBA, Ltd. was used (glass electrode method) .

The gold content of the suspension L was 0.0114% by mass relative to the total mass of the suspension.

(2-2-3) Preparation of Suspension of Gold Nanoplates

(Suspension M)

A suspension of gold nanoplates (suspension M) was prepared in the same manner as in the suspension of gold nanoplates (suspension K) except that the above-described amount of 10-fold diluted solution of gold-nanoplate seed particle suspension added was changed from 0.031 g to 0.062 g. The CTAC concentration in the dispersion medium of the suspension M was calculated to be 47.1 mM.

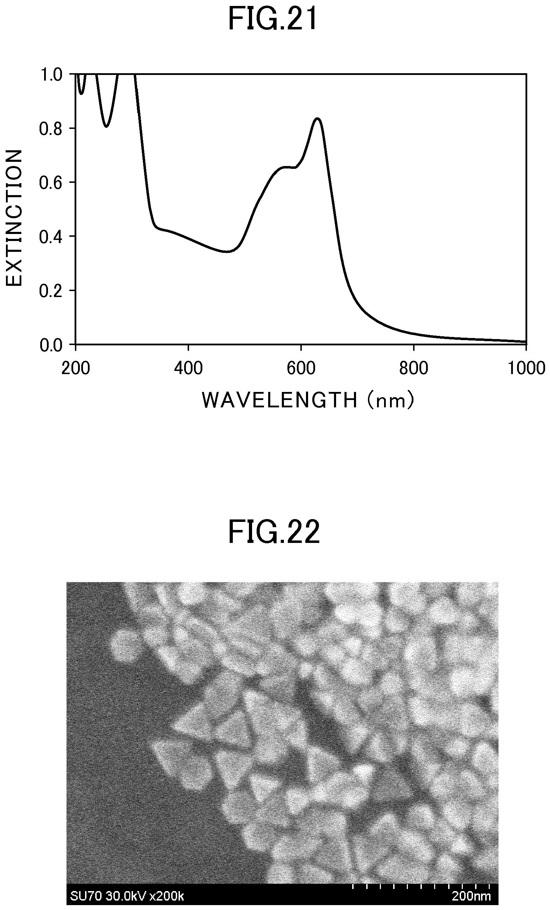

When the suspension M was 4-fold diluted by volume with ultrapure water, the color tone of the suspension was blue. Fig. 21 shows the optical properties of this 4-fold diluted suspension. The wavelength exhibiting the maximum absorbance was 628 nm (extinction: 0.84) . Since the extinction at 540 nm being the maximum absorption wavelength of non-plate-shaped polygonal or spherical gold nanoparticles was 0.59, the purity index of gold nanoplates was calculated to be 0.70. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 22 shows the results of scanning electron microscope (SEM) observation of the gold nanoplates in the obtained suspension M with an ultra-high-resolution analytical scanning electron microscope SU-70 (manufactured by Hitachi, Ltd.). In addition, the pH of the suspension M as a result of measurement at room temperature (20°C) was 2.4. For the pH measurement, a Twin pH Meter B-212 manufactured by HORIBA, Ltd. was used (glass electrode method) .

The gold content of the suspension M was 0.0114% by mass relative to the total mass of the suspension.

(2-2-4) Preparation of Suspension of Gold Nanoplates

(Suspension N)

A suspension of gold nanoplates (suspension N) was prepared in the same manner as in the suspension of gold nanoplates (suspension K) except that the above-described amount of 10-fold diluted solution of gold-nanoplate seed particle suspension added was changed from 0.031 g to 0.155 g. The CTAC concentration in the dispersion medium of the suspension N was calculated to be 47.0 mM.

When the suspension N was 4-fold diluted by volume with ultrapure water, the color tone of the suspension was purple.

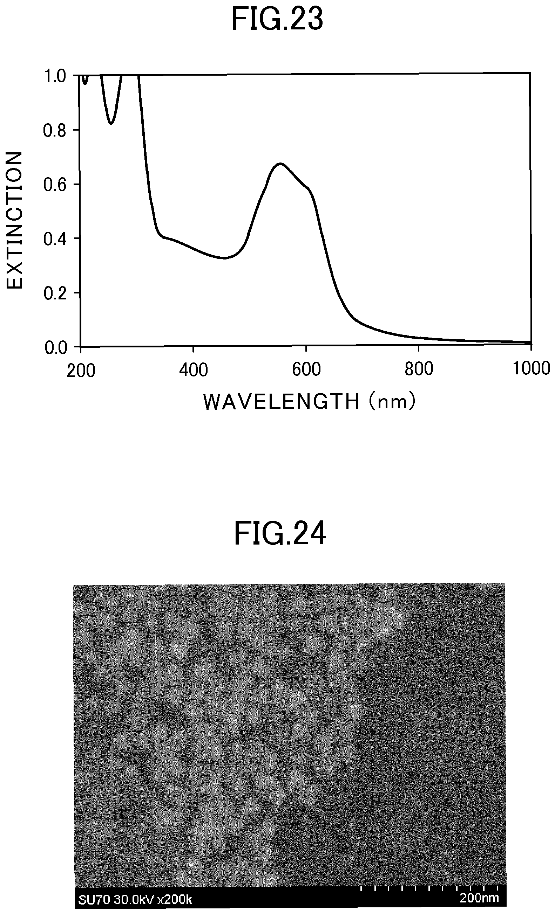

Fig. 23 shows the optical properties of this 4-fold diluted suspension. In the extinction spectrum, the position of a peak present on the long-wavelength side of the wavelength exhibiting the maximum absorbance (peak originating from the gold nanoplates) was 602 nm (extinction: 0.58). Since the extinction at 540 nm being the maximum absorption wavelength of non-plate-shaped polygonal or spherical gold nanoparticles was 0.64, the purity index of gold nanoplates was calculated to be 1.10. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 24 shows the results of scanning electron microscope (SEM) observation of the gold nanoplates in the obtained suspension N with an ultra-high-resolution analytical scanning electron microscope SU-70 (manufactured by Hitachi, Ltd.). In addition, the pH of the suspension N as a result of measurement at room temperature (20°C) was 2.4. For the pH measurement, a Twin pH Meter B-212 manufactured by HORIBA, Ltd. was used (glass electrode method).

The gold content of the suspension N was 0.0114% by mass relative to the total mass of the suspension.

(3) Purification of Suspension

(3-1) Purification of Suspension A (Preparation of Suspension B)

Into 30 g of the suspension A, 4.0 g of 1.0 M aqueous solution of CTAC was injected and stirred. The supernatant was removed while left standing at 30°C for 12 hours. The precipitate was dispersed with 20 g of 50 mM aqueous solution of CTAC to prepare a purified product of the suspension A (suspension B). The CTAC concentration of the suspension B in the dispersion medium was assumed to be 50 mM (CTAC concentration in the dispersion medium used at the time of redispersion).

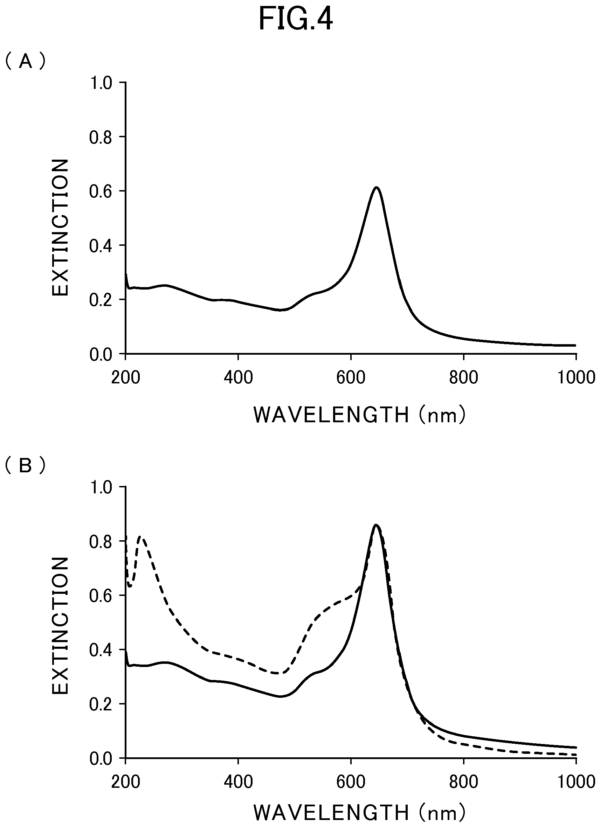

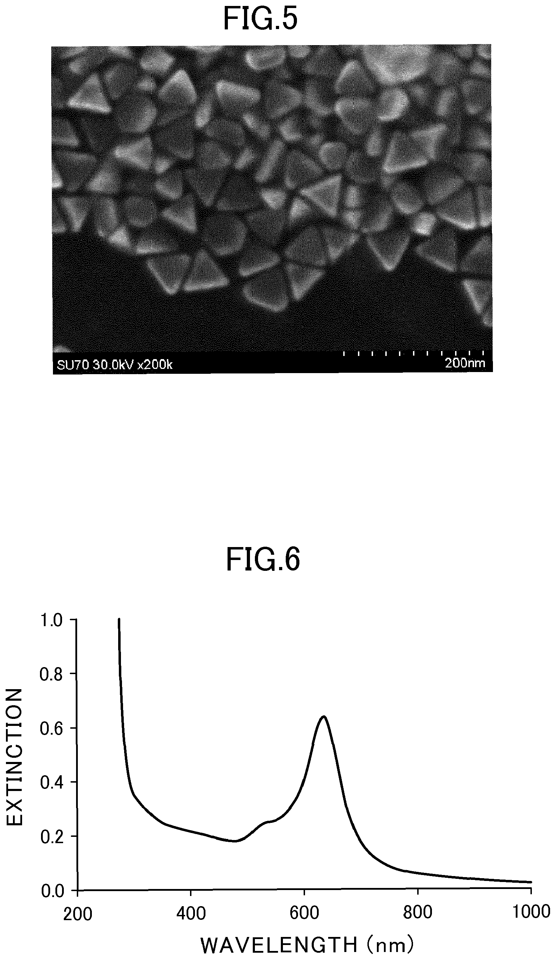

When the suspension B was 4-fold diluted by volume with ultrapure water, the color tone of the suspension was cyan tone. Fig. 4A shows the optical properties of this 4-fold diluted suspension. The wavelength exhibiting the maximum absorbance was 644 nm (extinction: 0.61) . Since the extinction at 540 nm being the maximum absorption wavelength of non-plate-shaped polygonal or spherical gold nanoparticles was 0.22, the purity index of gold nanoplates was calculated to be 0.36. From the extinction ratio, the estimated value of the gold content of the suspension B was 0.0079% by mass relative to the total mass of the suspension. Fig. 4B shows comparison between the extinction spectrum of the above-described suspension A (dashed line) and the extinction spectrum of the suspension B (solid line) in which all extinctions in the measurement wavelength region are multiplied by 1.41 to match the heights of extinctions at the maximum absorption wavelength. In the extinction spectrum of the suspension B, the extinction at 540 nm being the maximum absorption wavelength of non-plate-shaped polygonal or spherical gold nanoparticles was reduced by 39%. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 5 shows the results of scanning electron microscope (SEM) observation of the gold nanoplates in the obtained suspension B with an ultra-high-resolution analytical scanning electron microscope SU-70 (manufactured by Hitachi, Ltd.). SEM observation revealed that the gold nanoplates had a maximum length of 52 nm, a thickness of 19 nm, and an aspect ratio of 2.7.

The zeta potential of the gold nanoplates in the suspension B was +41.4 ± 1.2. The zeta potential was measured with Photal ELS-Z manufactured by Otsuka Electronics Co., Ltd.

In addition, when the precipitate of the above-described suspension A was dispersed with 0.02 g of 50 mM aqueous solution of CTAC to prepare a purified product of the suspension A (suspension W), the estimated value of the gold content was 7.9% by mass relative to the total mass of the suspension.

(3-2) Purification of Suspension K (Preparation of Suspension O)

Into 30 g of the suspension K, 4.0 g of 1.0 M aqueous solution of CTAC was injected and stirred. The supernatant was removed while left standing at 30°C for 12 hours. The precipitate was dispersed with 20 g of 50 mM aqueous solution of CTAC to prepare a purified product of the suspension K (suspension O). The CTAC concentration of the suspension O in the dispersion medium was assumed to be 50 mM.

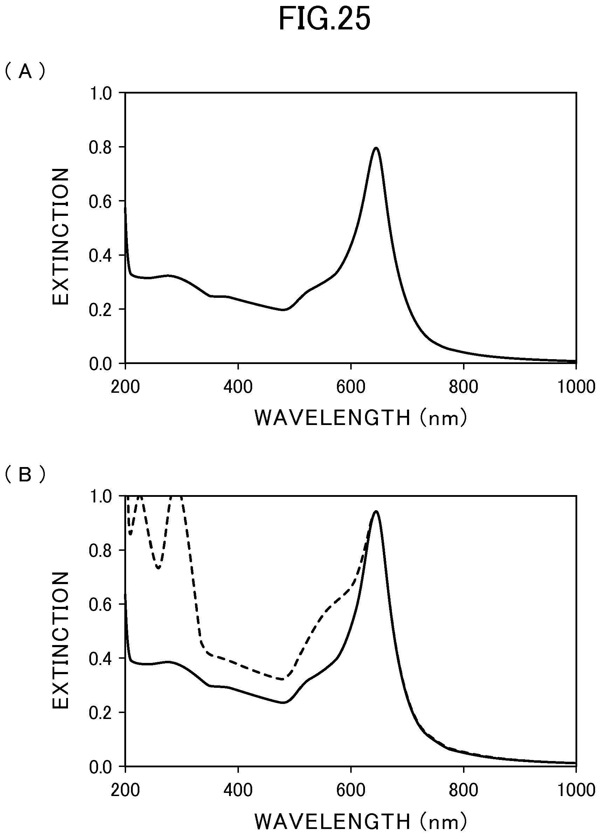

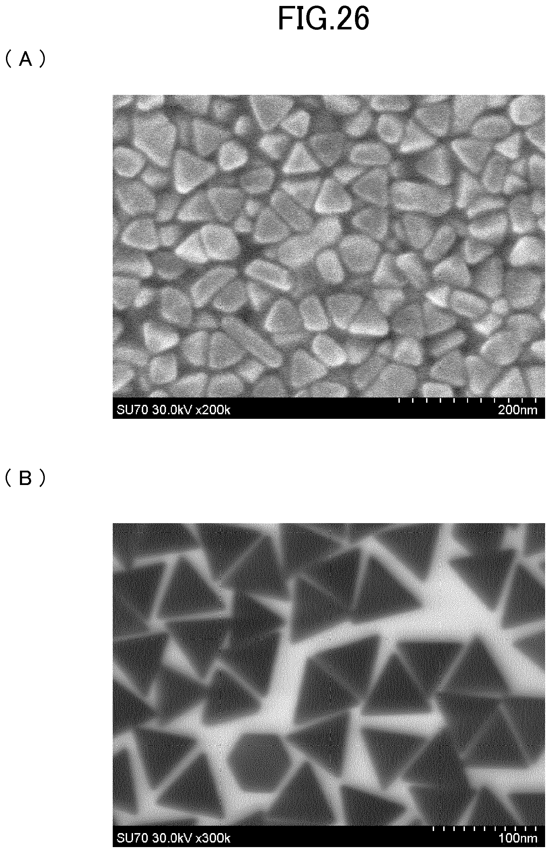

When the suspension O was 4-fold diluted by volume with ultrapure water, the color tone of the suspension was cyan tone. Fig. 25A shows the optical properties of this 4-fold diluted suspension. The wavelength exhibiting the maximum absorbance was 644 nm (extinction: 0.80) . Since the extinction at 540 nm being the maximum absorption wavelength of non-plate-shaped polygonal or spherical gold nanoparticles was 0.29, the purity index of gold nanoplates was calculated to be 0.36. From the extinction ratio, the estimated value of the gold content of the suspension O was 0.0085% by mass relative to the total mass of the suspension. Fig. 25B shows comparison between the extinction spectrum of the above-described suspension K (dashed line) and the extinction spectrum of the suspension O (solid line) in which all extinctions in the measurement wavelength region are multiplied by 1.18 to match the heights of extinctions at the maximum absorption wavelength. In the extinction spectrum of the suspension O, the extinction at 540 nm being the maximum absorption wavelength of non-plate-shaped polygonal or spherical gold nanoparticles was reduced by 36%. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 26A shows the results of scanning electron microscope (SEM) observation of the gold nanoplates in the obtained suspension O with an ultra-high-resolution analytical scanning electron microscope SU-70 (manufactured by Hitachi, Ltd.). Fig. 26B shows the results of adding a thiol-terminated polyethylene glycol (Mw: 20, 000) to the suspension O in order to observe a clear image, followed by scanning transmission electron microscope (STEM) observation. SEM and STEM observation revealed that the gold nanoplates had a maximum length of 65 nm, a thickness of 31 nm, and an aspect ratio of 2.1.

The zeta potential of the gold nanoplates in the suspension O was +42.0 ± 1.5. The zeta potential was measured with Photal ELS-Z manufactured by Otsuka Electronics Co., Ltd.

(3-3) Purification of Suspension L (Preparation of Suspension P)

Into 30 g of the suspension L, a 1.0 M aqueous solution of CTAC was injected and stirred. The amount injected was such an amount that the supernatant of the suspension L turned reddish purple. The reddish purple supernatant was removed while left standing at 30°C for 12 hours. The precipitate was dispersed with 30 g of 50 mM aqueous solution of CTAC to prepare a purified product of the suspension L (suspension P). The CTAC concentration of the suspension P in the dispersion medium was assumed to be 50 mM.

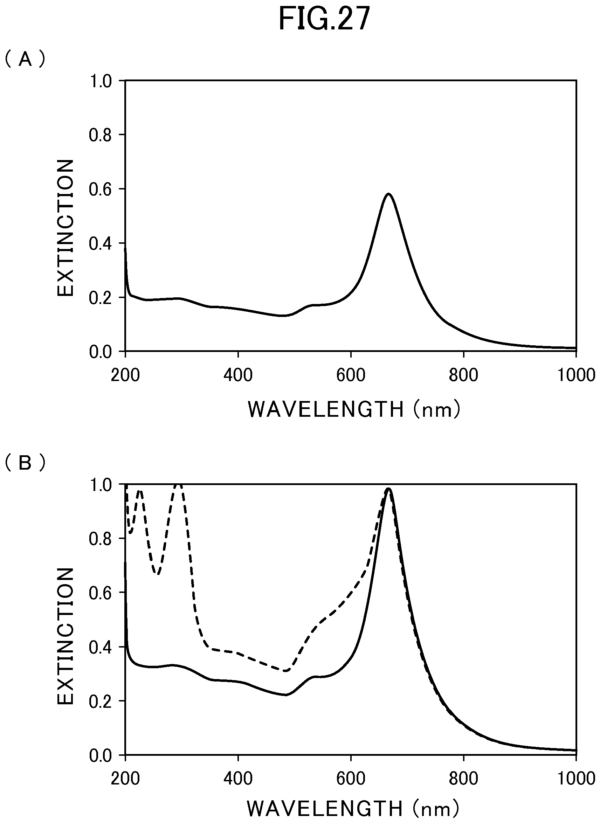

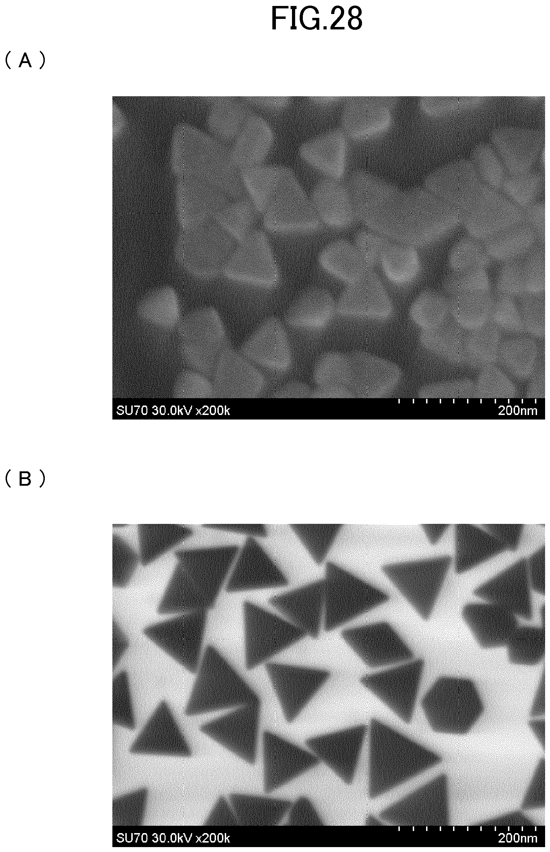

When the suspension P was 4-fold diluted by volume with ultrapure water, the color tone of the suspension was pale blue tone. Fig. 27A shows the optical properties of this 4-fold diluted suspension. The wavelength exhibiting the maximum absorbance was 668 nm (extinction: 0.58) . Since the extinction at 540 nm being the maximum absorption wavelength of non-plate-shaped polygonal or spherical gold nanoparticles was 0.17, the purity index of gold nanoplates was calculated to be 0.29. From the extinction ratio, the estimated value of the gold content of the suspension P was 0.0059% by mass relative to the total mass of the suspension. Fig. 27B shows comparison between the extinction spectrum of the above-described suspension L (dashed line) and the extinction spectrum of the suspension P (solid line) in which all extinctions in the measurement wavelength region are multiplied by 1.70 to match the heights of extinctions at the maximum absorption wavelength. In the extinction spectrum of the suspension P, the extinction at 540 nm being the maximum absorption wavelength of non-plate-shaped polygonal or spherical gold nanoparticles was reduced by 40%. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 28A shows the results of scanning electron microscope (SEM) observation of the gold nanoplates in the obtained suspension with an ultra-high-resolution analytical scanning electron microscope SU-70 (manufactured by Hitachi, Ltd.). Fig. 28B shows the results of adding a thiol-terminated polyethylene glycol (Mw: 20,000) to the suspension P in order to observe a clear image, followed by scanning transmission electron microscope (STEM) observation. SEM and STEM observation revealed that the gold nanoplates had a maximum length of 91 nm, a thickness of 41 nm, and an aspect ratio of 2.2.

The zeta potential of the gold nanoplates in the suspension P was +41.6 $\pm$ 2.0. The zeta potential was measured with Photal ELS-Z manufactured by Otsuka Electronics Co., Ltd.

(3-4) Purification of Suspension M (Preparation of Suspension Q)

Into 30 g of the suspension M, a 1.0 M aqueous solution of CTAC was injected and stirred. The amount injected was such an amount that the supernatant of the suspension M turned reddish purple. The reddish purple supernatant was removed while left standing at 30°C for 12 hours. The precipitate was dispersed with 30 g of 50 mM aqueous solution of CTAC to prepare a purified product of the suspension M (suspension Q). The CTAC concentration of the suspension Q in the dispersion medium was assumed to be 50 mM.

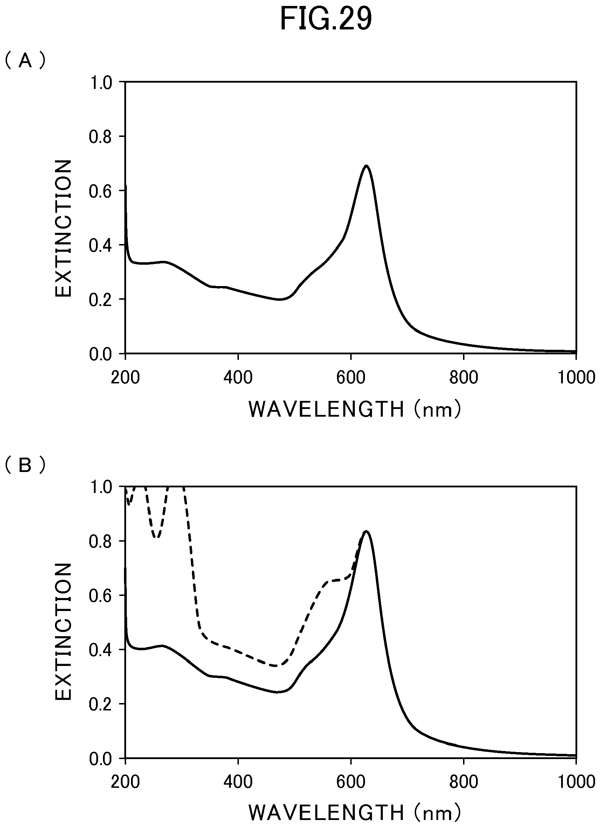

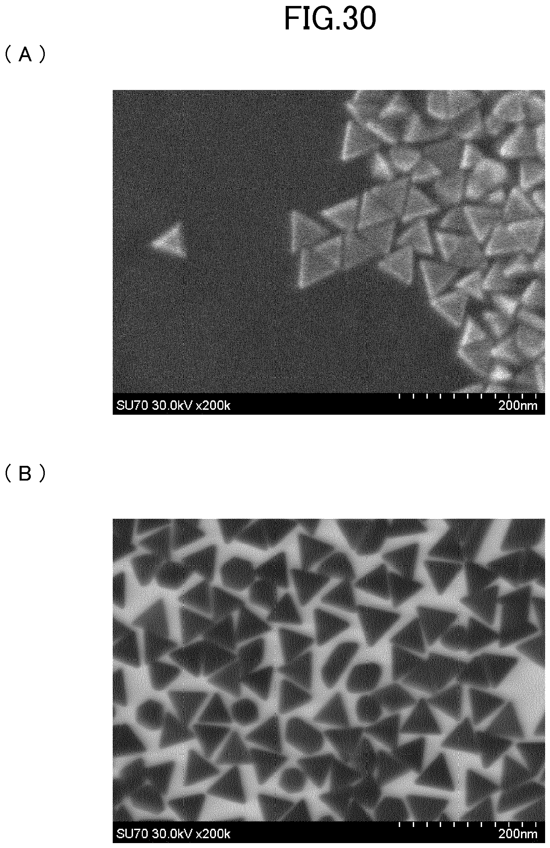

When the suspension Q was 4-fold diluted by volume with ultrapure water, the color tone of the suspension was pale blue tone. Fig. 29A shows the optical properties of this 4-fold diluted suspension. The wavelength exhibiting the maximum absorbance was 628 nm (extinction: 0.69). Since the extinction at 540 nm being the maximum absorption wavelength of non-plate-shaped polygonal or spherical gold nanoparticles was 0.31, the purity index of gold nanoplates was calculated to be 0.45. From the extinction ratio, the estimated value of the gold content of the suspension Q was 0.0080% by mass relative to the total mass of the suspension. Fig. 29B shows comparison between the extinction spectrum of the above-described suspension M (dashed line) and the extinction spectrum of the suspension Q (solid line) in which all extinctions in the measurement wavelength region are multiplied by 1.22 to match the heights of extinctions at the maximum absorption wavelength. In the extinction spectrum of the suspension Q, the extinction at 540 nm being the maximum absorption wavelength of non-plate-shaped polygonal or spherical gold nanoparticles was reduced by 36%. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 30A shows the results of scanning electron microscope (SEM) observation of the gold nanoplates in the obtained suspension with an ultra-high-resolution analytical scanning electron microscope SU-70 (manufactured by Hitachi, Ltd.). Fig. 30B shows the results of adding a thiol-terminated polyethylene glycol (Mw: 20,000) to the suspension Q in order to observe a clear image, followed by scanning transmission electron microscope (STEM) observation. SEM and STEM observation revealed that the gold nanoplates had a maximum length of 54 nm, a thickness of 27 nm, and an aspect ratio of 2.0.

The zeta potential of the gold nanoplates in the suspension Q was +42.1 $\pm$ 1.0. The zeta potential was measured with Photal ELS-Z manufactured by Otsuka Electronics Co., Ltd.

(3-5) Purification of Suspension N (Preparation of Suspension R)

Into 30 g of the suspension N, a 1.0 M aqueous solution of CTAC was injected and stirred. The amount injected was such an amount that the supernatant of the suspension N turned reddish purple. The reddish purple supernatant was removed while left standing at 30°C for 12 hours. The precipitate was dispersed with 30 g of 50 mM aqueous solution of CTAC to prepare a purified product of the suspension N (suspension R). The CTAC concentration of the suspension R in the dispersion medium was assumed to be 50 mM.

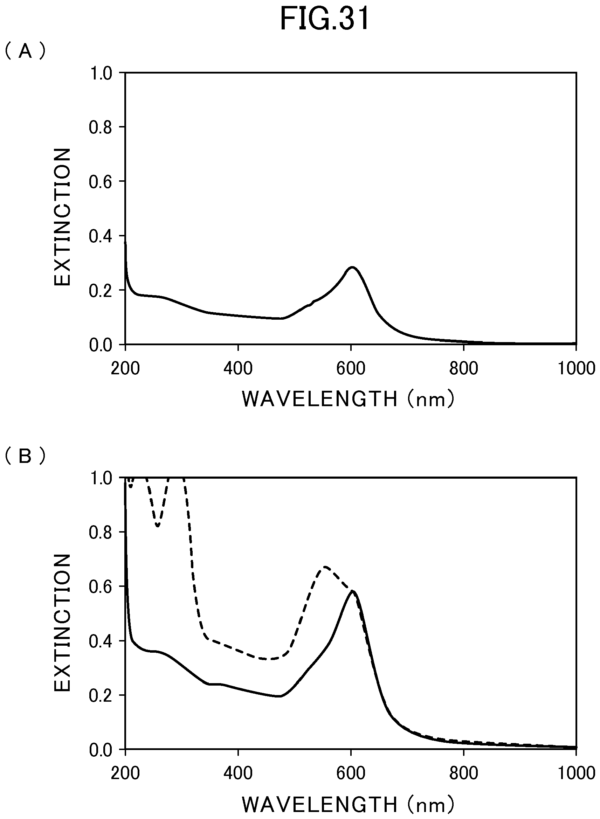

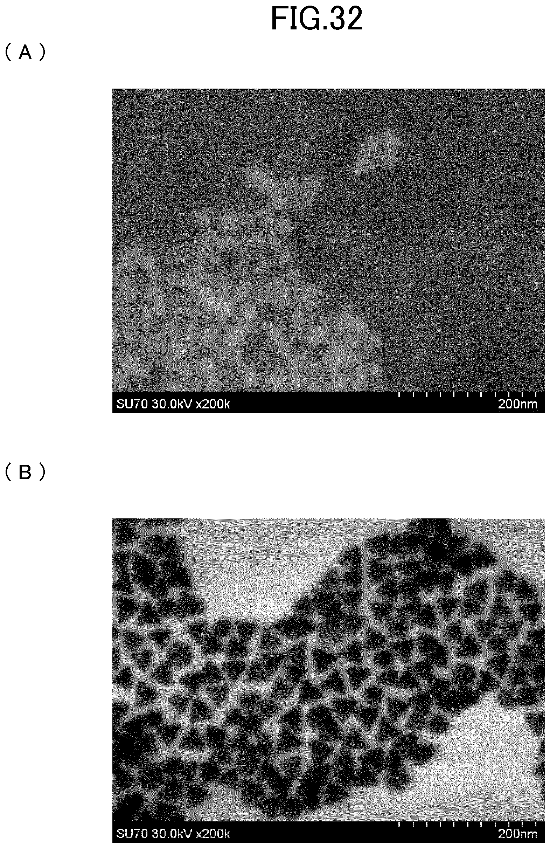

When the suspension R was 4-fold diluted by volume with ultrapure water, the color tone of the suspension was pale blue tone. Fig. 31A shows the optical properties of this 4-fold diluted suspension. The wavelength exhibiting the maximum absorbance was 602 nm (extinction: 0.28) . Since the extinction at 540 nm being the maximum absorption wavelength of non-plate-shaped polygonal or spherical gold nanoparticles was 0.16, the purity index of gold nanoplates was calculated to be 0.57. From the extinction ratio, the estimated value of the gold content of the suspension R was 0.0041% by mass relative to the total mass of the suspension. Fig. 31B shows comparison between the extinction spectrum of the above-described suspension N (dashed line) and the extinction spectrum of the suspension R (solid line) in which all extinctions in the measurement wavelength region are multiplied by 2.06 to match the heights of extinctions at the maximum absorption wavelength. In the extinction spectrum of the suspension R, the extinction at 540 nm being the maximum absorption wavelength of non-plate-shaped polygonal or spherical gold nanoparticles was reduced by 48%. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. Fig. 32A shows the results of scanning electron microscope (SEM) observation of the gold nanoplates in the obtained suspension with an ultra-high-resolution analytical scanning electron microscope SU-70 (manufactured by Hitachi, Ltd.). Fig. 32B shows the results of adding a thiol-terminated polyethylene glycol (Mw: 20,000) to the suspension R in order to observe a clear image, followed by scanning transmission electron microscope (STEM) observation. SEM and STEM observation revealed that the gold nanoplates had a maximum length of 37 nm, a thickness of 19 nm, and an aspect ratio of 1.9.

The zeta potential of the gold nanoplates in the suspension R was +41.1 ± 1.8. The zeta potential was measured with Photal ELS-Z manufactured by Otsuka Electronics Co., Ltd. (4) Preparation of Suspension of PSS-Coated Gold Nanoplates (Suspensions C to E) (Reduction of CTAC Concentration in Dispersion Medium of Suspension B)

Centrifugation (8,000 × g, 30°C, 10 minutes) was carried out on 40 mL of the suspension B prepared in (3) described above, followed by precipitation of the gold nanoplates to remove 39.6 mL of the supernatant. After that, the gold nanoplates at the bottom portion of the centrifuge tube was added with 39.6 mL of 20 $\mu$M aqueous solution of poly(sodium p-styrenesulfonate) (Na-PSS) for redispersion to prepare a suspension of PSS-coated gold nanoplate intermediate 1 (suspension C). The CTAC concentration of the suspension C in the dispersion medium was calculated to be 0.50 mM, and the PSS concentration was assumed to be 20 $\mu$M (PSS concentration in the dispersion medium used at the time of redispersion) . The zeta potential of the gold nanoplates in the suspension C was -51.0 ± 0.7. The zeta potential was measured with Photal ELS-Z manufactured by Otsuka Electronics Co., Ltd.

Centrifugation (8,000 × g, 30°C, 10 minutes) was carried out on the suspension C, followed by precipitation of the PSS-coated gold nanoplate intermediate 1 to remove 39.6 mL of the supernatant. After that, the PSS-coated gold nanoplate intermediate 1 at the bottom portion of the centrifuge tube was added with 39.6 mL of 20 $\mu$M aqueous solution of PSS for redispersion to prepare a suspension of PSS-coated gold nanoplate intermediate 2 (suspension D). The CTAC concentration of the suspension D in the dispersion medium was calculated to be $0.50 \times 10^{-2}$ mM, and the PSS concentration was assumed to be 20 $\mu$M. The zeta potential of the gold nanoplates in the suspension D was -51.0 ± 0.4. The zeta potential was measured with Photal ELS-Z manufactured by Otsuka Electronics Co., Ltd. Centrifugation (8,000 × g, 30°C, 10 minutes) was carried out on the suspension D, followed by precipitation of the PSS-coated gold nanoplate intermediate 2 to remove 39.6 mL of the supernatant. After that, the PSS-coated gold nanoplate intermediate 2 was added with 39.6 mL of 20 $\mu$M aqueous solution of PSS for redispersion to prepare a suspension of PSS-coated gold nanoplate (suspension E). The CTAC concentration of the suspension E in the dispersion medium was calculated to be $0.50 \times 10^{-4}$ mM, and the PSS concentration was assumed to be 20 $\mu$M. The zeta potential of the gold nanoplates in the suspension E was -54.5 ± 2.0. The zeta potential was measured with Photal ELS-Z manufactured by Otsuka Electronics Co., Ltd. The pH of the suspension of PSS-coated gold nanoplates as a result of measurement at room temperature (20°C) was 7.0. For the pH measurement, a Twin pH Meter B-212 manufactured by HORIBA, Ltd. was used (glass electrode method).

When the suspension E was 4-fold diluted by volume with ultrapure water, the color tone of the suspension was cyan tone. Fig. 6 shows the optical properties of this 4-fold diluted suspension. The wavelength exhibiting the maximum absorbance was 638nm (extinction: 0.64). Since the extinction at 540 nm being the maximum absorption wavelength of non-plate-shaped polygonal or spherical gold nanoparticles was 0.25, the purity index of gold nanoplates was calculated to be 0.39. From the extinction ratio, the estimated value of the gold content of the suspension E was 0.0085% by mass relative to the total mass of the suspension. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm.

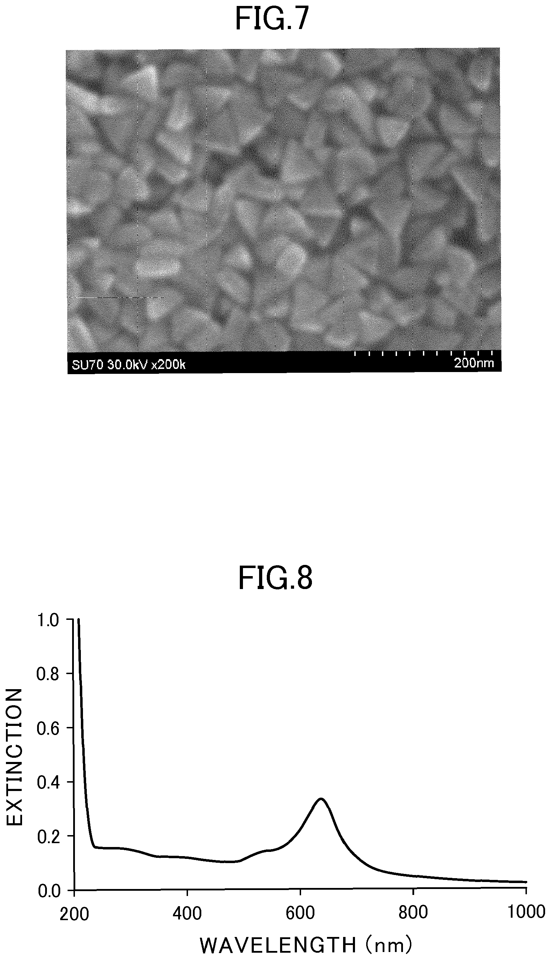

Fig. 7 shows the results of scanning electron microscope (SEM) observation of the gold nanoplates in the suspension E with an ultra-high-resolution analytical scanning electron microscope SU-70 (manufactured by Hitachi, Ltd.). SEM observation revealed that the gold nanoplates had a maximum length of 53 nm, a thickness of 19 nm, and an aspect ratio of 2.8.

(5) Preparation of Suspension of Citric Acid-Coated Gold Nanoplates

(5-1) Preparation of Suspension of Citric Acid-Coated Gold Nanoplates (Suspensions F to H) (Reduction of CTAC Concentration and Reduction of PSS Concentration in Aqueous Suspension E of PSS-Coated Gold Nanoplates)

Centrifugation (8,000 $\times$ g, 30°C, 10 minutes) was carried out on 40 mL of the aqueous suspension of PSS-coated gold nanoplates (suspension E) prepared in (4) described above, followed by precipitation of the PSS-coated gold nanoplates to remove 39.6 mL of the supernatant. After that, the PSS-coated gold nanoplates at the bottom portion of the centrifuge tube was added with 39.6 mL of 4.5 mM aqueous solution of trisodium citrate for redispersion to prepare a suspension of citric acid-coated gold nanoplate intermediate 1 (suspension F). The CTAC concentration of the suspension F in the dispersion medium was calculated to be $0.50 \times 10^{-6}$ mM, and the PSS concentration was calculated to be 0.20 $\mu$M. The zeta potential of the gold nanoplates in the suspension F was -47.5 $\pm$ 1.0. The zeta potential was measured with Photal ELS-Z manufactured by Otsuka Electronics Co., Ltd. Centrifugation (8,000 $\times$ g, 30°C, 10 minutes) was carried out on the suspension F, followed by precipitation of the citric acid-coated gold nanoplate intermediate 1 to remove 39.6 mL of the supernatant. After that, the citric acid-coated gold nanoplate intermediate 1 at the bottom portion of the centrifuge tube was added with 39.6 mL of 4.5 mM aqueous solution of trisodium citrate for redispersion to prepare a suspension of citric acid-coated gold nanoplate intermediate 2 (suspension G). The CTAC concentration of the suspension G in the dispersion medium was calculated to be $0.50 \times 10^{-8}$ mM, and the PSS concentration was calculated to be $0.20 \times 10^{-2}$ $\mu$M. The zeta potential of the gold nanoplates in the suspension G was -44.2 $\pm$ 1.7. The zeta potential was measured with Photal ELS-Z manufactured by Otsuka Electronics Co., Ltd. Centrifugation (8,000 $\times$ g, 30°C, 10 minutes) was carried out on the suspension G, followed by precipitation of the citric acid-coated gold nanoplate intermediate 2 to remove 39.6 mL of the supernatant. After that, the citric acid-coated gold nanoplate intermediate 2 was added with 39.6 mL of 4.5 mM aqueous solution of citric acid for redispersion to prepare a suspension of citric acid-coated gold nanoplates (suspension H). The CTAC concentration of the suspension H in the dispersion medium was calculated to be $0.50 \times 10^{-10}$ mM, and the PSS concentration was calculated to be $0.20 \times 10^{-4}$ $\mu$M. The zeta potential of the gold nanoplates in the suspension H was -42.2 $\pm$ 1.4. The zeta potential was measured with Photal ELS-Z manufactured by Otsuka Electronics Co., Ltd. The pH of the suspension H as a result of measurement at room temperature (20°C) was pH 2.4. For the pH measurement, a Twin pH Meter B-212 manufactured by HORIBA, Ltd. was used (glass electrode method).

When the suspension H was 4-fold diluted by volume with ultrapure water, the color tone of the suspension was cyan tone. Fig. 8 shows the optical properties of this 4-fold diluted suspension. The wavelength exhibiting the maximum absorbance was 638 nm (extinction: 0.33) . Since the extinction at 540 nm being the maximum absorption wavelength of non-plate-shaped polygonal or spherical gold nanoparticles was 0.14, the purity index of gold nanoplates was calculated to be 0.42. From the extinction ratio, the estimated value of the gold content of the suspension H was 0.0045% by mass relative to the total mass of the suspension. The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm.

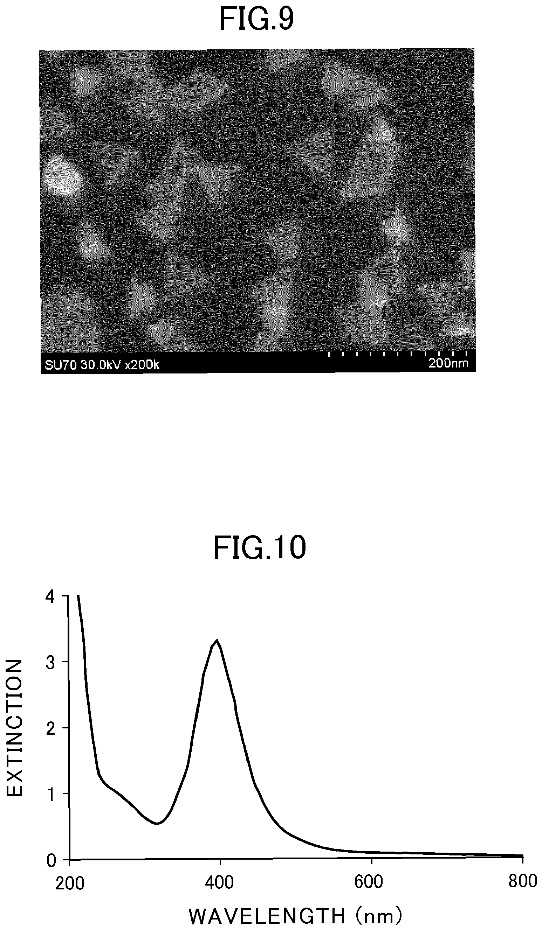

Fig. 9 shows the results of scanning electron microscope (SEM) observation of the gold nanoplates in the suspension H with an ultra-high-resolution analytical scanning electron microscope SU-70 (manufactured by Hitachi, Ltd.). SEM observation revealed that the gold nanoplates had a maximum length of 55 nm, a thickness of 19 nm, and an aspect ratio of 2.9.

(5-2) Preparation of Suspension of Citric Acid-Coated Gold Nanoplates (Suspensions S to V)

The above-described step of changing the suspension B of gold nanoplates to the suspension H was also carried out similarly on the suspensions O to R to prepare a suspension of citric acid-coated gold nanoplates (suspensions S to V). Suspension S

The CTAC concentration of the suspension S in the dispersion medium was calculated to be $0.50 \times 10^{-10}$ mM, and the PSS concentration was calculated to be $0.20 \times 10^{-4}$ $\mu$M. The zeta potential of the gold nanoplates in the suspension S was -41.5 $\pm$ 0.5. The pH of the suspension S as a result of measurement at room temperature (20°C) was pH 7.9. The color tone of the suspension where the suspension S was 4-fold diluted by volume with ultrapure water was cyan tone. The wavelength exhibiting the maximum absorbance was 634 nm (extinction: 0.32). Since the extinction at 540 nm being the maximum absorption wavelength of non-plate-shaped polygonal or spherical gold nanoparticles was 0.12, the purity index of gold nanoplates was calculated to be 0.38. From the extinction ratio, the estimated value of the gold content of the suspension S was 0.0034% by mass relative to the total mass of the suspension.

Suspension T

The CTAC concentration of the suspension T in the dispersion medium was calculated to be $0.50 \times 10^{-10}$ mM, and the PSS concentration was calculated to be $0.20 \times 10^{-4}$ μM. The zeta potential of the gold nanoplates in the suspension T was -42.2 $\pm$ 1.2. The pH of the suspension T as a result of measurement at room temperature (20°C) was pH 8.2. The color tone of the suspension where the suspension T was 4-fold diluted by volume with ultrapure water was pale blue tone. The wavelength exhibiting the maximum absorbance was 658 nm (extinction: 0.23) . Since the extinction at 540 nm being the maximum absorption wavelength of non-plate-shaped polygonal or spherical gold nanoparticles was 0.07, the purity index of gold nanoplates was calculated to be 0.30. From the extinction ratio, the estimated value of the gold content of the suspension T was 0.0023% by mass relative to the total mass of the suspension.

Suspension U

The CTAC concentration of the suspension U in the dispersion medium was calculated to be $0.50 \times 10^{-10}$ mM, and the PSS concentration was calculated to be $0.20 \times 10^{-4}$ μM. The zeta potential of the gold nanoplates in the suspension U was -41.7 $\pm$ 2.7. The pH of the suspension U as a result of measurement at room temperature (20°C) was pH 8.1. The color tone of the suspension where the suspension U was 4-fold diluted by volume with ultrapure water was blue tone. The wavelength exhibiting the maximum absorbance was 618 nm (extinction: 0.28). Since the extinction at 540 nm being the maximum absorption wavelength of non-plate-shaped polygonal or spherical gold nanoparticles was 0.13, the purity index of gold nanoplates was calculated to be 0.46. From the extinction ratio, the estimated value of the gold content of the suspension U was 0.0033% by mass relative to the total mass of the suspension.

Suspension V

The CTAC concentration of the suspension V in the dispersion medium was calculated to be $0.50 \times 10^{-10}$ mM, and the PSS concentration was calculated to be $0.20 \times 10^{-4}$ μM. The zeta potential of the gold nanoplates in the suspension V was -40.8 $\pm$ 1.5. The pH of the suspension V as a result of measurement at room temperature (20°C) was pH 8.2. The color tone of the suspension where the suspension V was 4-fold diluted by volume with ultrapure water was blue tone. The wavelength exhibiting the maximum absorbance was 592 nm (extinction: 0.11). Since the extinction at 540 nm being the maximum absorption wavelength of non-plate-shaped polygonal or spherical gold nanoparticles was 0.07, the purity index of gold nanoplates was calculated to be 0.64. From the extinction ratio, the estimated value of the gold content of the suspension V was 0.0017% by mass relative to the total mass of the suspension.

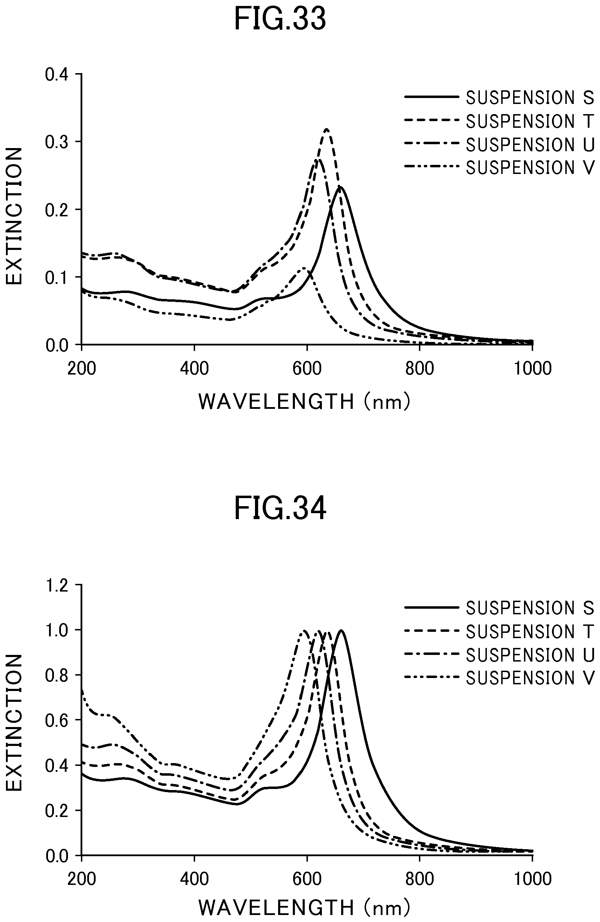

The zeta potentials of the suspensions S to V were measured with Photal ELS-Z manufactured by Otsuka Electronics Co., Ltd. For the pH measurement of the suspensions S to V, a Twin pH Meter B-212 manufactured by HORIBA, Ltd. was used (glass electrode method) . The optical properties of the suspensions S to V were measured using a UV-Vis-NIR spectrometer MPC 3100 UV-3100 PC manufactured by Shimadzu Corporation under conditions of an optical path length: 1 cm and a measurement wavelength: 190 to 1300 nm. In addition, Fig. 33 shows the optical properties of the suspensions of the suspensions S to V, and Fig. 34 shows the optical properties when the extinction at the maximum absorption wavelength is set to 1.0.

2. Suspension Containing Gold-Coated Silver Nanoplates

(1) Preparation of Silver-Nanoplate Seed Particles

To 20 mL of 2.5 mM aqueous solution of trisodium citrate, 1 mL of 0.5 g/L aqueous solution of polystyrene sulfonic acid having a molecular weight of 70,000 and 1.2 mL of 10 mM aqueous solution of sodium borohydride were added, followed by addition of 50 mL of 0.5 mM aqueous solution of silver nitrate at 20 mL/min with stirring. The obtained solution was left standing in an incubator (30°C) for 60 minutes to prepare a suspension of silver-nanoplate seed particles.

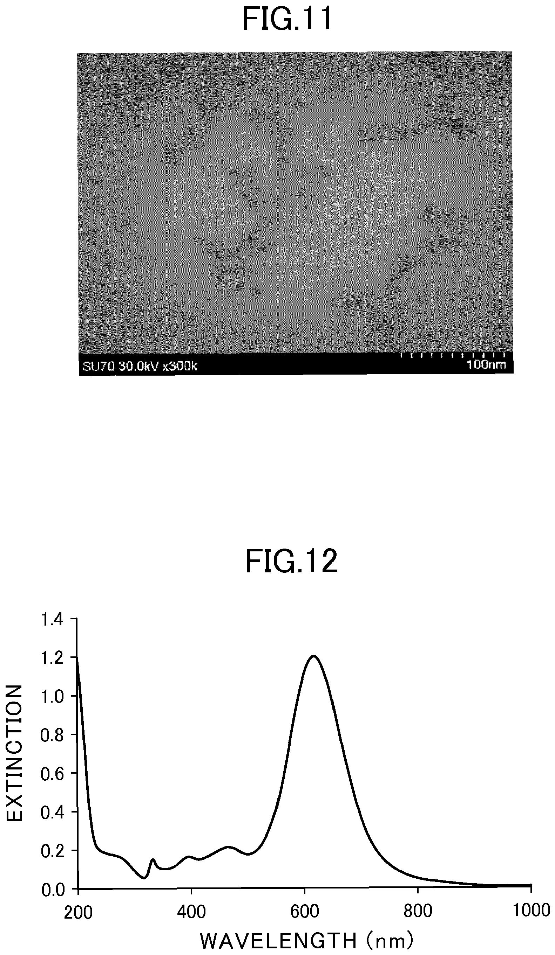

Fig. 10 shows the optical properties of the prepared suspension (raw solution). The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. The maximum absorption wavelength of the silver-nanoplate seed particles was 396 nm (extinction 3.3) corresponding to the maximum absorption wavelength originating from LSPR of the spherical silver nanoparticles. In this connection, the extinction of the present invention is the value of the absorbance when a suspension is measured with a spectrometer. In addition, Fig. 11 shows the results of scanning electron microscope (SEM) observation with an ultra-high-resolution analytical scanning electron microscope SU-70 (manufactured by Hitachi, Ltd.). SEM observation revealed that the particles had particle diameters of mainly 3 nm or more and less than 10 nm (aspect ratio was about 1.0) .

(2) Preparation of Silver Nanoplates

To 200 mL of ultrapure water, 4.5 mL of 10 mM aqueous solution of ascorbic acid was added and 2 mL of the suspension of silver-nanoplate seed particles prepared in (1) described above was further added. The obtained solution was added with 120 mL of 0.5 mM aqueous solution of silver nitrate at 30 mL/min with stirring. The stirring was stopped 4 minutes after the addition of the aqueous solution of silver nitrate was finished, followed by addition of 20 mL of 25 mM aqueous solution of trisodium citrate. The obtained solution was left standing in an incubator (30°C) in an air atmosphere for 100 hours to prepare a suspension of silver nanoplates.

Fig. 12 shows the optical properties of the suspension where the prepared suspension was 4-fold diluted by volume with ultrapure water. The wavelength exhibiting the maximum absorbance was 618 nm (extinction 1.20). The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm. SEM observation of the silver nanoplates in the suspension revealed that the silver nanoplates had an average particle diameter of 50 nm, an average thickness of 10 nm, and an aspect ratio of 5.0. An ultra-high-resolution analytical scanning electron microscope SU-70 manufactured by Hitachi, Ltd. was used for the analysis of the SEM observation image.

(3) Preparation of Gold-Coated Silver Nanoplates (Color Tone: Cyan)

To 120 mL of the suspension of silver nanoplates prepared in (2) described above, 9.1 mL of 0.125 mM aqueous solution of polyvinylpyrrolidone (PVP) (molecular weight: 40,000) was added, followed by addition of 1.6 mL of 0.5 M aqueous solution of ascorbic acid to add 9.6 mL of 0.14 mM aqueous solution of chloroauric acid at 0.5 mL/min with stirring. The obtained solution was left standing in an incubator (30°C) for 24 hours to prepare a suspension of gold-coated silver nanoplates in which the surfaces of the silver nanoplates were coated with gold.

The main dispersion medium of the suspension of gold-coated silver nanoplates was water. The gold-coated silver nanoplates contained in this suspension were a mixture of plates having circular shapes and polygonal shapes including triangular shapes in which an average maximum length (particle diameter) of the main surfaces was 50 nm, and had an average thickness of 10 nm. Moreover, an average thickness of gold on the gold-coated silver nanoplates was 0.30 nm.

The pH of the suspension of gold-coated silver nanoplates was 4.0 at room temperature (20°C). For the pH measurement, a Twin pH Meter B-212 manufactured by HORIBA, Ltd. was used (glass electrode method).

The silver content of the suspension of gold-coated silver nanoplates was 0.0016% by mass relative to the total mass of the suspension.

(4) pH Adjustment of Suspension of Gold-Coated Silver Nanoplates

To 1.95 mL of the suspension obtained in (3) described above, 0.05 mL of 200 mM phosphate buffer saline (not containing a divalent ion; hereinafter also referred to as "PBS (-) buffer solution" in some cases) and 0.025 mL of 190 mM aqueous solution of sodium carbonate were added with stirring to prepare a pH-adjusted suspension of gold-coated silver nanoplates (suspension I).

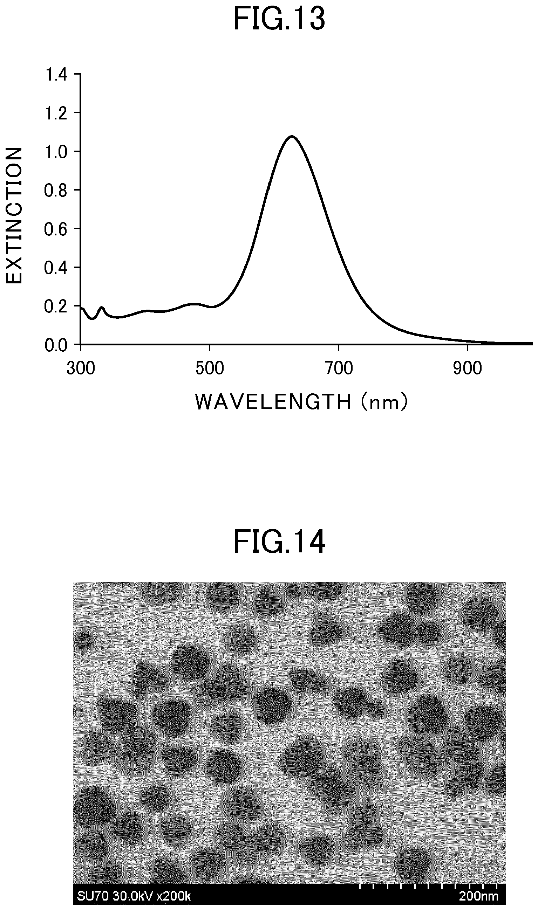

Fig. 13 shows the optical properties of the suspension being the suspension I 3.4-fold diluted by volume with ultrapure water. The wavelength exhibiting the maximum absorbance was 628 nm (extinction 1.07). The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm.

The main dispersion medium of the suspension I was water.

The gold-coated silver nanoplates contained in the suspension I were a mixture of plates having circular shapes and polygonal shapes including triangular shapes in which an average maximum length of the main surfaces was 50 nm, and had an average thickness of 10 nm. Moreover, an average thickness of gold on the gold-coated silver nanoplates contained in the suspension I was 0.30 nm. Fig. 14 shows the results of scanning electron microscope (SEM) observation with an ultra-high-resolution analytical scanning electron microscope SU-70 (manufactured by Hitachi, Ltd.).

The color tone of the 3-fold diluted solution of the suspension I was cyan.

The pH of the suspension I was 7.3 at room temperature (20°C).

The silver content of the suspension I was 0.0015% by mass relative to the total mass of the suspension. 3. Suspension of PVP-Stabilized Spherical Gold Nanoparticles (1) Preparation of Suspension of PVP-Stabilized Spherical Gold Nanoparticles

To 12 mL of commercially available spherical gold nanoparticles (particle diameter: 40 nm, concentration: 0.0068% by mass), 0.91 mL of 0.125 mM aqueous solution of polyvinylpyrrolidone (PVP) (molecular weight: 40,000) was added with stirring. The obtained solution was left standing in an incubator (30°C) for 24 hours to prepare a suspension of PVP-stabilized spherical gold nanoparticles.

(2) pH Adjustment of Suspension of PVP-Stabilized Spherical Gold Nanoparticles

To 1.95 mL of the suspension J obtained in (1), 0.05 mL of 200 mM PBS buffer solution and 0.025 mL of 190 mM aqueous solution of sodium carbonate were added with stirring to prepare a pH-adjusted suspension of PVP-stabilized spherical gold nanoparticles (suspension J).

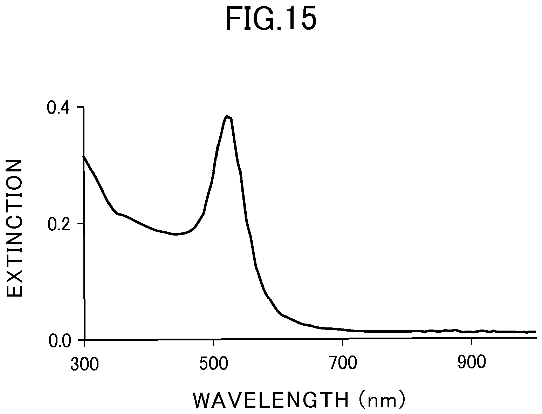

Fig. 15 shows the optical properties of the aqueous suspension being the prepared suspension J 3.8-fold diluted by volume with ultrapure water. The wavelength exhibiting the maximum absorbance was 522 nm (extinction 0.38). The optical properties were measured using a UV-Vis-NIR spectrometer MPC 3100 UV-3100 PC manufactured by Shimadzu Corporation under conditions of an optical path length: 1 cm and a measurement wavelength: 190 to 1300 nm.

The main dispersion medium of the suspension was water.

The spherical gold nanoparticles contained in the suspension J had an average particle diameter of 40 nm.

The color tone of the suspension J was red.

The pH of the suspension J was 7.0 at room temperature (20°C).

The gold content of the suspension J was 0.0061% by mass relative to the total mass of the suspension.

4. Immunochromatographic Test

Each of the suspensions of Examples and Comparative Examples was subjected to an immunochromatographic test according to the following procedure. The test result was evaluated.

(1) Preparation of Developing Solutions Used in Immunochromatographic Test (Supporting of Specific Binding Substance onto Each Suspension of Gold Nanoplates; labeling of detection reagent)

First, 0.2 mL of a solution of an antibody (product name: Goat anti HBsAg, manufactured by: Arista Biologicals, Inc.) (detection antibody in the immunochromatography) against a *hepatitis B virus* antigen (HBs antigen) at a concentration of 50 $\mu$g/mL in a 1 mM PBS (-) buffer solution was mixed with 1.8 mL of each suspension of gold nanoplates (the suspensions A to H and S to V). The obtained mixture was shaken at room temperature for 60 minutes. Then, 0.044 mL of a solution of SUNBRIGHT ME-020SH (molecular weight: 2,000, manufactured by NOF CORPORATION), which is a polyethylene glycol having an end modified with a thiol group, at a concentration of 0.4 mM in a 1 mM PBS (-) buffer solution was added. The obtained suspension was shaken at room temperature for 30 minutes . Then, 0.100 mL of a solution of bovine serum albumins (product name: Bovine Serum Albumin, abbreviated as: BSA, molecular weight: 66 kDa, manufactured by: Sigma-Aldrich) at a concentration of 0.5 mM in a 1 mM PBS (-) buffer solution was added. The obtained suspension was shaken at room temperature for 30 minutes . Then, centrifugation (8,000 $\times$ g, 30°C, 10 minutes) was carried out on this suspension, followed by precipitation of the antibody-gold nanoplate complex to remove 1.90 mL of supernatant. Subsequently, the antibody-gold nanoplate complex was added with 0.40 mL of 1 mM PBS (-) buffer solution (containing 4.9 $\mu$M of BSA) for redispersion. The resultant was adjusted to have an extinction of 1.0 by using a UV-Vis-NIR spectrometer MPC 3100 UV-3100 PC manufactured by Shimadzu Corporation. In this manner, developing solutions were prepared.

(2) Preparation of Developing Solutions Used in Immunochromatographic Test (Supporting of Specific Binding Substance onto Gold-Coated Silver Nanoplates (labeling of detection reagent))

First, 0.2 mL of a solution of an antibody (product name: Goat anti HBsAg, manufactured by: Arista Biologicals, Inc.) (detection antibody in the immunochromatography) against a *hepatitis B virus* antigen (HBs antigen) at a concentration of 50 $\mu$g/mL in a 1 mM PBS (-) buffer solution was mixed with 1.8 mL of the suspension of gold-coated silver nanoplates (the suspension I). The obtained mixture was shaken at room temperature for 60 minutes. Then, 0.044 mL of a solution of SUNBRIGHT ME-020SH (molecular weight: 2,000, manufactured by NOF CORPORATION), which is a polyethylene glycol having an end modified with a thiol group, at a concentration of 0.4 mM in a 1 mM PBS (-) buffer solution was added. The obtained suspension was shaken at room temperature for 30 minutes . Then, 0.100 mL of a solution of bovine serum albumins (product name: Bovine Serum Albumin, abbreviated as: BSA, molecular weight: 66 kDa, manufactured by: Sigma-Aldrich) at a concentration of 0.5 mM in a 1 mM PBS (-) buffer solution was added. The obtained suspension was shaken at room temperature for 30 minutes . Then, centrifugation (26,000 $\times$ g, 30°C, 10 minutes) was carried out on this suspension, followed by precipitation of the complex of the antibody and the gold-coated silver nanoplates to remove 1.90 mL of supernatant. Subsequently, the complex of the antibody and the gold-coated silver nanoplates was added with 1.90 mL of 1 mM PBS (-) buffer solution (containing 4.9 $\mu$M of BSA) for redispersion. The resultant was adjusted to have an extinction of 1.0 by using a UV-Vis-NIR spectrometer MPC 3100 UV-3100 PC manufactured by Shimadzu Corporation. In this manner, the developing solution I (color tone: cyan) was prepared.

(3) Preparation of Developing Solutions Used in Immunochromatographic Test (Supporting of Specific Binding Substance onto Spherical Gold Nanoparticles; labeling of detection reagent)

First, 0.2 mL of a solution of an antibody (product name: Goat anti HBsAg, manufactured by: Arista Biologicals, Inc.) (detection antibody in the immunochromatography) against a *hepatitis B virus* antigen (HBs antigen) at a concentration of 50 $\mu$g/mL in a 1 mM PBS (-) buffer solution was mixed with 1.8 mL of the suspension of PVP-stabilized spherical gold nanoparticles (the suspension J). The obtained mixture was shaken at room temperature for 60 minutes. Then, 0.044 mL of a solution of SUNBRIGHT ME-020SH (molecular weight: 2,000, manufactured by NOF CORPORATION), which is a polyethylene glycol having an end modified with a thiol group, at a concentration of 0.8 mM in a 1 mM PBS (-) buffer solution was added. The obtained suspension was shaken at room temperature for 30 minutes. Then, 0.100 mL of a solution of bovine serum albumins (product name: Bovine Serum Albumin, abbreviated as: BSA, molecular weight: 66 kDa, manufactured by: Sigma-Aldrich) at a concentration of 0.5 mM in a 1 mM PBS (-) buffer solution was added. The obtained suspension was shaken at room temperature for 30 minutes. Then, centrifugation (4200 $\times$ g, 30°C, 10 minutes) was carried out on this suspension, followed by precipitation of the complex of the antibody and the spherical gold nanoparticles to remove 1.90 mL of supernatant. Subsequently, the complex of the antibody and the spherical gold nanoparticles was added with 1.90 mL of 1 mM PBS (-) buffer solution (containing 4.9 $\mu$M of BSA) for redispersion. The resultant was adjusted to have an extinction of 1.0 by using a UV-Vis-NIR spectrometer MPC 3100 UV-3100 PC manufactured by Shimadzu Corporation. In this manner, the developing solution J (color tone: red) was prepared.

Table 1 below shows the relationship between the physical properties of the used suspensions of gold nanoplates and the prepared developing solutions. For the suspensions and the developing solutions, C to H and S to V correspond to Examples of the present invention, and A, B, I, and J correspond to Comparative Examples.

[Table 1]

Table 1: Relationships between physical properties of suspensions of gold nanoplates and prepared developing solutions

| Suspension | Metal Type | Shape | Color Tone | CTAC (mM) | PSS ($\mu$M) | PVP ($\mu$M) | Developing Solution |
|---|---|---|---|---|---|---|---|
| A | Gold | Plate | Blue Tone | 48.0 | 0 | 0 | A |
| B | Gold | Plate | Cyan Tone | 50 | 0 | 0 | B |
| C | PSS-Coated Gold | Plate | Cyan Tone | 0.50 | 20 | 0 | C |
| D | PSS-Coated Gold | Plate | Cyan Tone | $0.50 \times 10^{-2}$ | 20 | 0 | D |
| E | PSS-Coated Gold | Plate | Cyan Tone | $0.50 \times 10^{-4}$ | 20 | 0 | E |
| F | Citric Acid-Coated Gold | Plate | Cyan Tone | $0.50 \times 10^{-6}$ | 0.2 | 0 | F |
| G | Citric Acid-Coated Gold | Plate | Cyan Tone | $0.50 \times 10^{-8}$ | $0.2 \times 10^{-2}$ | 0 | G |
| H | Citric Acid-Coated Gold | Plate | Cyan Tone | $0.50 \times 10^{-10}$ | $0.2 \times 10^{-4}$ | 0 | H |
| S | Citric Acid-Coated Gold | Plate | Cyan Tone | $0.50 \times 10^{-10}$ | $0.2 \times 10^{-4}$ | 0 | S |
| T | Citric Acid-Coated Gold | Plate | Pale Blue Tone | $0.50 \times 10^{-10}$ | $0.2 \times 10^{-4}$ | 0 | T |

(continued)

| Suspension | Metal Type | Shape | Color Tone | CTAC (mM) | PSS ($\mu$M) | PVP ($\mu$M) | Developing Solution |
|---|---|---|---|---|---|---|---|
| U | Citric Acid-Coated Gold | Plate | Blue Tone | $0.50 \times 10^{-10}$ | $0.2 \times 10^{-4}$ | 0 | U |
| V | Citric Acid-Coated Gold | Plate | Blue Tone | $0.50 \times 10^{-10}$ | $0.2 \times 10^{-4}$ | 0 | V |
| I | Gold-Coated Silver | Plate | Cyan Tone | 0 | 0 | 7.8 | I |
| J | Gold | Spherical | Red Tone | 0 | 0 | 8.6 | J |

(4) Immunochromatographic Test

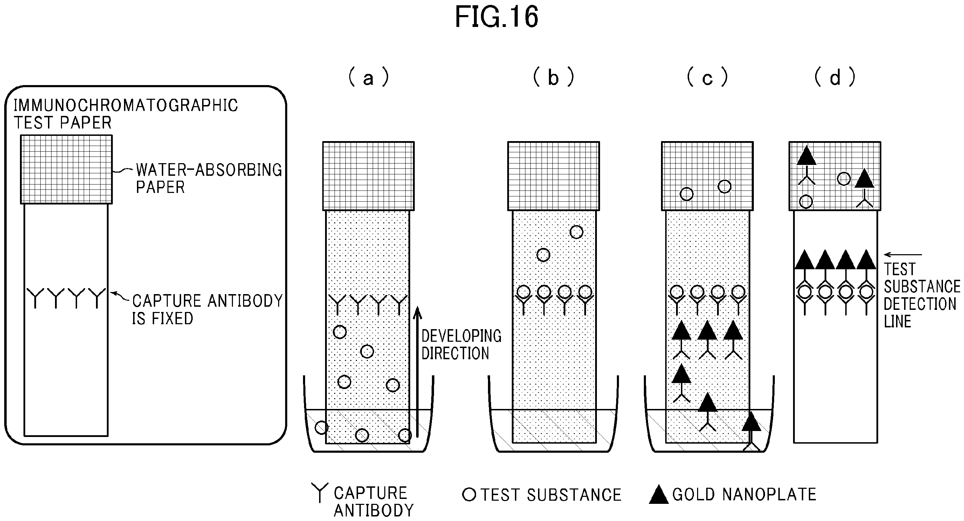

According to the procedure illustrated in Fig. 16, an immunochromatographic test with the test substance of a *hepatitis B virus* antigen (HBs antigen) was conducted using an immunochromatographic test paper having an anti-HBs antibody (capture antibody) immobilized in a straight line (the detection line of Fig. 16).

The immunochromatographic test paper used was purchased from a contractor company (BioDevice Technology, Co., Ltd.) for preparing immunochromatographic test papers. When the capture antibody was immobilized in a straight line, the capture antibody used was adjusted to a concentration of 1 mg/mL by using a 5 mM PBS (-) buffer solution.

A first developing solution (developing solution used in Fig. 16(a)) was a solution of an HBs antigen (product name: HBsAg Protein (Subtype adr), manufactured by: Fitzgerald Industries International Inc.) in a 1 mM PBS (-) buffer solution. As the first developing solution, solutions were prepared which had HBs antigen concentrations of 6.0 $\mu$M, 0.6 $\mu$M, 0.06 $\mu$M, 0.006 $\mu$M, and 0 M (blank), respectively.

A second developing solution was a 1 mM PBS (-) buffer solution.

A third developing solution (developing solution used in Fig. 16(c)) was the above-described developing solutions A to J.

The specific test procedure was as follows.

On the immunochromatographic test paper, 15 $\mu$L of the first developing solution was developed (Fig. 16(a)). As the first developing solution was developed, the HBs antigen was captured by the capture antibody immobilized on the detection line of the test paper (Fig. 16(b)).

Then, 30 $\mu$L of the second developing solution was developed to wash away excessive antigens on the immunochromatographic test paper.

Finally, 60 $\mu$L of the third developing solution was developed (Fig. 16(c)). As the third developing solution was developed, the detection antibody (such as the complex of the antibody and the PSS-coated gold nanoplates) bound to the HBs antigen captured on the detection line of the test paper (Fig. 16(d)).

The same procedure was repeated for each of the first developing solutions having different HBs concentrations.

(5) Immunochromatographic Test Evaluation by Visual Judgment

After the third developing solution was developed, the coloring of the metal nanoparticles on the detection line was visually checked to judge the presence or absence of the HBs antigen. Table 2 below shows the result.

[Table 2]

Table 2: HBs antigen concentration in the first developing solution (µM) and results of visual judgment

| Developing Solution | | A | B | C | D | E | F | G | H | S | T | U | V | I | J |
|---|---|---|---|---|---|---|---|---|---|---|---|---|---|---|---|
| Antigen (µM) | 6 | - | - | + | + | + | + | ++ | ++ | ++ | ++ | ++ | ++ | + | + |
| | 0.6 | - | - | + | + | + | + | + | + | + | + | + | + | + | + |
| | 0.06 | - | - | + | + | + | + | + | + | + | + | + | + | + | + |
| | 0.006 | - | - | + | + | + | + | + | + | + | + | + | + | + | + |
| 0 (Blank) | | - | - | - | - | - | - | - | - | - | - | - | - | - | - |

| ++: Deep coloring on the detection line was successfully confirmed. |
| +: Coloring on the detection line was successfully confirmed. |
| -: Coloring on the detection line was not confirmed. |

(6) Immunochromatographic Test Evaluation by Luminance Analysis

After the third developing solution was developed, the immunochromatographic test paper was scanned (apparatus name: Cano Scan LiDE500F, manufactured by: Canon Inc.) to quantify the lowest luminance at the detection line (immobilized section of the capture antibody) and the lowest luminance at a section other than the detection line (control region) by using image analysis software (Image-J: open-source, public-domain image processing software (http://imagej.nih.gov/ij/) developed by Wayne Rasband at the National Institutes of Health). The lowest luminance was determined by measuring once luminances at five different positions in the detection line or the control region and employing a median of the obtained numerical values as the lowest luminance. The luminance difference was calculated according to (the lowest luminance at the detection line - the lowest luminance at the control region). Table 3 shows the result.

[Table 3]

Table 3: HBs antigen concentration in the first developing solution (µM) and results of luminance analysis

| Developing Solution | | A | B | C | D | E | F | G | H | S | T | U | V | I | J |
|---|---|---|---|---|---|---|---|---|---|---|---|---|---|---|---|
| Antigen (µM) | 6 | 0 | 0 | 80 | 73 | 73 | 75 | 85 | 90 | 91 | 85 | 95 | 94 | 80 | 73 |
| | 0.6 | 0 | 0 | 44 | 43 | 40 | 40 | 45 | 49 | 49 | 46 | 53 | 55 | 44 | 43 |
| | 0.06 | 0 | 0 | 21 | 25 | 20 | 20 | 34 | 40 | 42 | 32 | 44 | 44 | 21 | 25 |
| | 0.006 | 0 | 0 | 4 | 9 | 9 | 0 | 11 | 15 | 16 | 10 | 17 | 16 | 4 | 9 |
| 0 (Blank) | | 0 | 0 | 0 | 0 | 0 | 0 | 0 | 0 | 0 | 0 | 0 | 0 | 0 | 0 |

The values in the entries for each developing solution represent luminance differences.

(7) Dry Resistance Test After Immunochromatographic Test

With the concentration of HBs antigens in the first developing solution being 6.0 µM, the immunochromatographic test paper after the development of the third developing solution was dried in a room at a temperature of 20°C and a humidity of 50%. The immunochromatographic test paper was scanned (apparatus name: Cano Scan LiDE500F, manufactured by: Canon Inc.) when the drying was started (0 hours) and when 24 hours, 48 hours, 168 hours, 720 hours, 720 hours, and 2160 hours had passed since the start of the drying. The obtained image was imported to Adobe Photoshop, bitmap image editing software, to measure CIELAB D50 of the detection line and the target region (the detection line or a section other than the detection line) . The color difference between the detection line and the target region (∆E) was calculated from formulas 1 below. (Formulas 1) Color Difference Calculation Formulas

$$\Delta E = \sqrt{(\Delta L^2 + \Delta a^2 + \Delta b^2)}$$

$$\Delta L = L_1 - L_2$$

$$\Delta a = a_1 - a_2$$

$$\Delta b = b_1 - b_2$$

(In the Lab color space being a type of complementary color space, the L-value represents lightness, and the a-value and the b-value represent the strength of tint. A positive and a negative a-value represent red hue and green hue, respectively. A positive and a negative b-value represent yellow hue and violet hue, respectively. In formulas 1, $L_1$ means the L-value of the detection line, $L_2$ the L-value of the control region, $a_1$ the a-value of the detection line, $a_2$ the a-value of the control region, $b_1$ the b-value of the detection line, and $b_2$ the b-value of the control region.)

Table 4 below shows the results of color difference calculation.

[Table 4]

Table 4: Drying time for immunochromatography paper and results of color difference measurement

| Developing Solution | | C | D | E | F | G | H | S | T | U | V | I | J |
|---|---|---|---|---|---|---|---|---|---|---|---|---|---|
| Drying Time | 0 hours | 70 | 72 | 75 | 80 | 86 | 91 | 91 | 85 | 95 | 94 | 80 | 70 |
| | 24 hours | 55 | 58 | 60 | 67 | 72 | 76 | 77 | 73 | 80 | 79 | 46 | 50 |
| | 48 hours | 47 | 49 | 53 | 59 | 64 | 67 | 69 | 69 | 75 | 73 | 30 | 40 |
| | 168 hours | 46 | 47 | 52 | 58 | 63 | 66 | N.D | N.D | N.D. | N.D. | 23 | 35 |
| | 720 hours | 44 | 45 | 50 | 55 | 60 | 63 | N.D. | N.D. | N.D. | N.D | 15 | 32 |
| N.D.: No data. | | | | | | | | | | | | | |

In the immunochromatographic test, no coloring of the detection line was observed or no luminance difference was produced (the data is now shown) even when the third developing solution containing gold nanoplates not supporting an antibody was used. However, when the third developing solution containing gold nanoplates supporting an antibody originating from suspensions C to H or S to V was used, a clear line capable of visual judgment was observed in a manner similar to the case of using the third developing solution containing gold-coated silver nanoplates supporting an antibody or spherical gold nanoparticles supporting an antibody. Also, a significant luminance difference was measured. The detection line rapidly appeared particularly when the gold nanoplates were used. On the other hand, when the third developing solution containing gold nanoplates supporting an antibody originating from the suspensions A and B was used, no coloring of the detection line was observed or no luminance difference was produced. It is believed that this is because CTAC inhibited the supporting of the antibody onto the gold nanoplates. To be more specific, when the hexadecyltrimethylammonium cations of CTAC form a double layer on the surfaces of the gold nanoplates, these cations cover the uppermost surfaces of the gold nanoplates. When the zeta potential becomes positive, the gold nanoplates and the antibody repel each other because the antibody also has a positive charge. Thus, the supporting of the antibody onto the gold nanoplates is inhibited. On the other hand, treatment using PSS being a polyanion-based polymer, followed by treatment with citric acid reduces the concentration of CTAC in the composition, and when the gold nanoplates are covered with these substances, the zeta potential of the gold nanoplates becomes negative, making it possible to support the antibody on the gold nanoplates.

Results similar to those of the immunochromatographic test of Examples were successfully obtained also in an immunochromatographic test targeting other test substances such as human chorionic gonadotropin (hCG). For this reason, it is understood that the advantages of the present invention should not be limited to a particular embodiment.

The above revealed that when gold nanoplates are prepared such that the average aspect ratio of the gold nanoplates (the value obtained by dividing the maximum length of the gold nanoplates by the thickness) is in a range greater than 1 and 10 or less and a composition is prepared such that the concentration of quaternary ammonium cations represented by $N^+(R)_4$ (each R is independently selected from linear or branched alkyl groups having 1 to 20 carbon atoms) is 1 mM or less, it is possible to provide a composition which has a maximum absorption wavelength in the visible light region and which contains gold nanoplates capable of easily supporting a specific binding substance for a test substance such as an antibody.

**Claims**

1. A composition comprising gold nanoplates, for detecting a test substance, wherein
   an average aspect ratio of the gold nanoplates (a value obtained by dividing a maximum length of the gold nanoplates by a thickness thereof) is greater than 1 and 10 or less,
   a concentration of quaternary ammonium cations in the composition is 0 to 1 mM, and
   the quaternary ammonium cations are represented by $N^+(R)_4$ (each R is independently selected from linear or branched alkyl groups having 1 to 20 carbon atoms).

2. The composition of claim 1, wherein
   the quaternary ammonium cations are selected from the group consisting of decyltrimethylammonium cations, hexadecyltrimethylammonium cations, and heptadecyltrimethylammonium cations.

3. The composition of claim 1 or 2, not containing a water-soluble polymer having a molecular weight of 500 or more.

4. The composition of claim 1 or 2, further comprising a water-soluble polymer having a molecular weight of 500 or more, wherein a concentration of the water-soluble polymer having the molecular weight of 500 or more in the composition is greater than 0 and 1% by mass or less.

5. The composition of claim 1 or 2, further comprising a water-soluble polymer having a molecular weight of 500 or more, wherein a concentration of the water-soluble polymer having the molecular weight of 500 or more in the composition is greater than 0 and 100 $\mu$M or less.

6. The composition of claim 4 or 5, wherein the gold nanoplates and the water-soluble polymer form a complex.

7. The composition of any one of claims 4 to 6, wherein the water-soluble polymer is selected from the group consisting of poly(sodium styrenesulfonate), polyvinylpyrrolidone, and thiol-terminated polyethylene glycol.

8. The composition of any one of claims 1 to 7, not containing sodium citrate.

9. The composition of any one of claims 1 to 7, further comprising sodium citrate, wherein a concentration of the sodium citrate in the composition is greater than 0 and 50 mM or less.

10. The composition of any one of claims 1 to 9, wherein an average maximum length of the gold nanoplates is less than 100 nm.

11. The composition of any one of claims 1 to 10, wherein the gold nanoplates have a light absorption peak attributed to surface plasmon resonance in a wavelength region of 550 to 740 nm.

12. The composition of any one of claims 1 to 11, exhibiting a color tone of red, magenta, purple, deep blue, blue, cyan, or pale blue.

13. The composition of any one of claims 1 to 12, wherein the gold nanoplates support a specific binding substance for the test substance.

14. The composition of claim 13, wherein a combination of the test substance and the specific binding substance, respectively, is selected from the group consisting of an antigen and an antibody capable of binding thereto, an antibody and an antigen capable of binding thereto, a sugar chain or a glycoconjugate and a lectin capable of binding to the sugar chain or the glycoconjugate, a lectin and a sugar chain or a glycoconjugate capable of binding to the lectin, a hormone or a cytokine and a receptor capable of binding to the hormone or the cytokine, a receptor and a hormone or a cytokine capable of binding to the receptor, a protein and a nucleic acid aptamer or a peptide aptamer capable of binding to the protein, an enzyme and a substrate capable of binding thereto, a substrate and an enzyme capable of binding thereto, biotin and avidin or streptavidin, avidin or streptavidin and biotin, IgG and Protein A or Protein G, Protein A or Protein G and IgG, and a first nucleic acid and a second nucleic acid capable of binding thereto.

**15.** A freeze-dried product of the composition of any one of claims 1 to 14, for detecting a test substance.

**16.** A method for detecting the test substance by using the composition of claim 13 or 14 or the freeze-dried product of claim 15, the method comprising the steps of:

mixing the composition or a resuspension of the freeze-dried product with the test substance to form a complex of the test substance with the gold nanoplates supporting the specific binding substance; and
detecting the complex.

**17.** The method of claim 16, wherein formation of the complex is detected by means selected from the group consisting of extinction measurement, absorbance measurement, turbidity measurement, particle size distribution measurement, particle diameter measurement, Raman scattering measurement, color-tone change observation, aggregate- or precipitate-formation observation, immunochromatography, electrophoresis, and flow cytometry.

**18.** The method of claim 16 or 17, further comprising the steps of:

preparing the composition of claim 13 or 14 by allowing the gold nanoplates in the composition of any one of claims 1 to 12 to support the specific binding substance for the test substance; and/or resuspending the freeze-dried product of claim 15.

**19.** A kit for use in the method of any one of claims 16 to 18, comprising the composition of any one of claims 1 to 14 or the freeze-dried product of claim 15.

**20.** A method for producing the composition of any one of claims 1 to 14 or the freeze-dried product of claim 15, comprising the steps of:

(1) preparing a suspension of gold-nanoplate seed particles; and
(2) preparing a suspension of gold nanoplates.

**21.** The method of claim 20, wherein the step (1) does not include a step of pre-growing gold-nanoplate seed particles.

# FIG.1

# FIG.2

# FIG.3

SU70 30.0kV x200k                                          200nm

# FIG.4

（A）

（B）

# FIG.5

SU70 30.0kV x200k                                    200nm

# FIG.6

# FIG.7

SU70 30.0kV x200k                                    200nm

# FIG.8

## FIG.9

SU70 30.0kV x200k                    200nm

## FIG.10

# FIG.11

SU70 30.0kV x300k                                    100nm

# FIG.12

# FIG.13

# FIG.14

SU70 30.0kV x200k                    200nm

# FIG.15

# FIG.16

IMMUNOCHROMATOGRAPHIC TEST PAPER

WATER-ABSORBING PAPER

CAPTURE ANTIBODY IS FIXED

(a)  (b)  (c)  (d)

DEVELOPING DIRECTION

TEST SUBSTANCE DETECTION LINE

Y CAPTURE ANTIBODY

○ TEST SUBSTANCE

▲ GOLD NANOPLATE

## FIG.17

## FIG.18

# FIG.19

# FIG.20

SU70 30.0kV x200k          200nm

# FIG.21

# FIG.22

## FIG.23

## FIG.24

# FIG.25

（A）

（B）

# FIG.26

（A）

（B）

# FIG.27

(A)

(B)

# FIG.28

（A）

SU70 30.0kV x200k    200nm

（B）

SU70 30.0kV x200k    200nm

# FIG.29

（A）

（B）

# FIG.30

（A）

（B）

# FIG.31

(A)

(B)

# FIG.32

（A）

SU70 30.0kV x200k　　　　　　200nm

（B）

SU70 30.0kV x200k　　　　　　200nm

# FIG.33

# FIG.34

## INTERNATIONAL SEARCH REPORT

| International application No. |
| --- |
| PCT/JP2017/009290 |

A.  CLASSIFICATION OF SUBJECT MATTER
*G01N33/553*(2006.01)i, *G01N33/543*(2006.01)i

According to International Patent Classification (IPC) or to both national classification and IPC

B.  FIELDS SEARCHED

Minimum documentation searched (classification system followed by classification symbols)
G01N33/553, G01N33/543

Documentation searched other than minimum documentation to the extent that such documents are included in the fields searched
Jitsuyo Shinan Koho       1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X | JP 2015-194495 A  (Dai Nippon Toryo Co., Ltd.), | 1-2,4-7, |
|  | 05 November 2015 (05.11.2015), | 9-14,16-20 |
| Y | paragraphs [0018] to [0028], [0045] to [0062] | 15 |
| A | (Family: none) | 3,8,21 |
|  |  |  |
| Y | JP 2007-303997 A  (Canon Inc.), | 15 |
| A | 22 November 2007 (22.11.2007), | 1-14,16-21 |
|  | paragraphs [0055] to [0066] |  |
|  | (Family: none) |  |
|  |  |  |
| A | HUANG, Y. et al., Surfactant-Promoted Reductive Synthesis of Shape-Controlled Gold Nanostructures, CRYSTAL GROWTH & DESIGN, 2009. 02.04, Vol. 9, No. 2, p. 858-862 | 1-21 |

☒  Further documents are listed in the continuation of Box C.          ☐   See patent family annex.

| * | Special categories of cited documents: |
| --- | --- |
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 26 April 2017 (26.04.17) | 16 May 2017 (16.05.17) |

| Name and mailing address of the ISA/ | Authorized officer |
| --- | --- |
| Japan Patent Office | |
| 3-4-3,Kasumigaseki,Chiyoda-ku, | |
| Tokyo 100-8915,Japan | Telephone No. |

Form PCT/ISA/210 (second sheet) (January 2015)

**INTERNATIONAL SEARCH REPORT**

International application No.

PCT/JP2017/009290

C (Continuation).   DOCUMENTS CONSIDERED TO BE RELEVANT

| Category* | Citation of document, with indication, where appropriate, of the relevant passages | Relevant to claim No. |
|---|---|---|
| A | HUANG, Y. et al., High-yield synthesis of triangular gold nanoplates with improved shape uniformity, tunable edge length and thickness, Nanoscale, 2014.04.11, Vol. 6, p. 6496-6500 | 1-21 |
| A | TANGEYSH, B. et al., Triangular Gold Nanoplate Growth by Oriented Attachment of Au Seeds Generated by Strong Field Laser Reduction, Nano letters, 2015.04.06, Vol. 15, p. 3377-3382 | 1-21 |
| A | CHU, H.C. et al., Thermal Aqueous Solution Approach for the Synthesis of Triangular and Hexagonal Gold Nanoplates with Three Different Size Ranges, Inorganic Chemistry, 2006.12.16, Vol. 45, No. 2, p. 808-813 | 1-21 |
| A | JP 2006-037221 A  (Korea Research Institute of Standards and Science), 09 February 2006 (09.02.2006), entire text; all drawings & US 2006/0021468 A1    & EP 1623781 A2 | 1-21 |

Form PCT/ISA/210 (continuation of second sheet) (January 2015)

**EP 3 428 646 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015522828 W
- JP 4428568 B
- JP 2008545884 W
- WO 2006099312 A

### Non-patent literature cited in the description

- **CHEN L. et al.** *Nano Letters,* 2014, vol. 14 (12), 7201-7206
- **SCARABELLI L et al.** *ACS Nano,* 2014, vol. 8 (6), 5833-5842
- *Nano Lett.,* 2004, vol. 4 (9), 1627-1631